(19) Europäisches Patentamt
European Patent Office
Office européen des brevets

(11) EP 2 028 185 A1

(12) EUROPÄISCHE PATENTANMELDUNG

(43) Veröffentlichungstag:
**25.02.2009 Patentblatt 2009/09**

(21) Anmeldenummer: **08018687.7**

(22) Anmeldetag: **20.10.2003**

(51) Int Cl.:
***C07K 5/02*** (2006.01) ***C07D 207/26*** (2006.01)
***C07D 277/56*** (2006.01) ***C07D 417/12*** (2006.01)
***C07F 7/18*** (2006.01) ***C07C 271/22*** (2006.01)
***C07D 211/60*** (2006.01) ***A61K 38/08*** (2006.01)

(84) Benannte Vertragsstaaten:
**AT BE BG CH CY CZ DE DK EE ES FI FR GB GR HU IE IT LI LU MC NL PT RO SE SI SK TR**

(30) Priorität: **21.11.2002 DE 10254439**

(62) Dokumentnummer(n) der früheren Anmeldung(en) nach Art. 76 EPÜ:
**03775204.5 / 1 562 979**

(71) Anmelder: **Helmholtz-Zentrum für Infektionsforschung GmbH**
**38124 Braunschweig (DE)**

(72) Erfinder:
- **Höfle, Gerhard**
**38124 Braunschweig (DE)**
- **Glaser, Nicole**
**38124 Braunschweig (DE)**
- **Steinmetz, Heinrich**
**38124 Braunschweig (DE)**
- **Leibold, Thomas**
**38124 Braunschweig (DE)**
- **Sasse, Florenz**
**38124 Braunschweig (DE)**

(74) Vertreter: **Forstmeyer, Dietmar et al**
**BOETERS & LIECK**
**Oberanger 32**
**80331 München (DE)**

Bemerkungen:
Diese Anmeldung ist am 24-10-2008 als Teilanmeldung zu der unter INID-Code 62 erwähnten Anmeldung eingereicht worden.

# (54) Tubulysine und Tubulysin-Mittel

(57) Die Erfindung betrifft eine Verbindung der folgenden allgemeinen Formel (Tubulysin) mit den folgenden Bedeutungen für R, $R^1$, $R^2$, $R^3$, $R^4$, $R^5$, $R^6$, $R^7$,$R^8$ $R^9$, R1^0, $R^{11}$, S, T, U, V, W, X, Y und Z: R = H, $C_{1-4}$-Alkyl, Aryl, $OR^1$, $NR^1R^2$ oder NH- $(CH_2)_{2}$-$R^1$ = H, $C_{1-6}$-Alkyl oder Aryl $R^2$ = H, $C_{1-6}$-Alkyl oder Aryl S = H, Hal, $NO_2$ oder $NHR^3$ U = H, Hal, $NO_2$ oder $NHR^3$ $R^3$ = H, HCO oder $C_{1-4}$-Alkyl-CO T = H oder $OR^4$ $R^4$ = H, $C_{1-4}$-Alkyl, Aryl, $COR^5$, P (O) $(OR^6)_2$ oder $SO_3R^6$ $R^5$ = $C_{1-6}$-Alkyl, Alkenyl, Aryl oder Heteroaryl $R^6$ = H, $C_{1-4}$-Alkyl oder Metallion V = H oder $R^7$ oder (für W = O) O $R^7$ = H, $C_{1-4}$-Alkyl oder $COR^8$ $R^8$ = C-$_{1-4}$-Alkyl, Alkenyl oder Aryl W = H oder $C_{1-4}$-Alkyl oder (für V = O) O X = H, $C_{1-4}$-Alkyl, Alkenyl oder $CH_20R^9$ $R^9$ = H, $C_{1-4}$-Alkyl, Alkenyl, Aryl oder $COR^{10}$ $R^{10}$ = $C_{1-6}$-Alkyl, Alkenyl, Aryl oder Heteroaryl Y = (für Z = $CH_3$ oder $COR^{11}$)freies Elektronenpaar oder (für Z =CH3) O $R^{11}$ = $C_{1-4}$-Alkyl, $CF_3$ oder Aryl und/oder Z = (für Y = O oder freies Elektronenpaar) $CH_3$ oder (für Y = freies Elektronenpaar) $COR^{11}$.

EP 2 028 185 A1

## Beschreibung

Tubulysine sind als Verbindungen der folgenden allgemeinen Formel bekannt; vgl. beispielsweise F. Sasse, H. Steinmetz, J. Heil, G. Höfle, H. Reichenbach, J. Antibiot.2000, 53, 579-558, und H. Reichenbach, G. Höfle, F. Sasse, H. Steinmetz (GBF), DE 196 38 870 A1, **1996.**

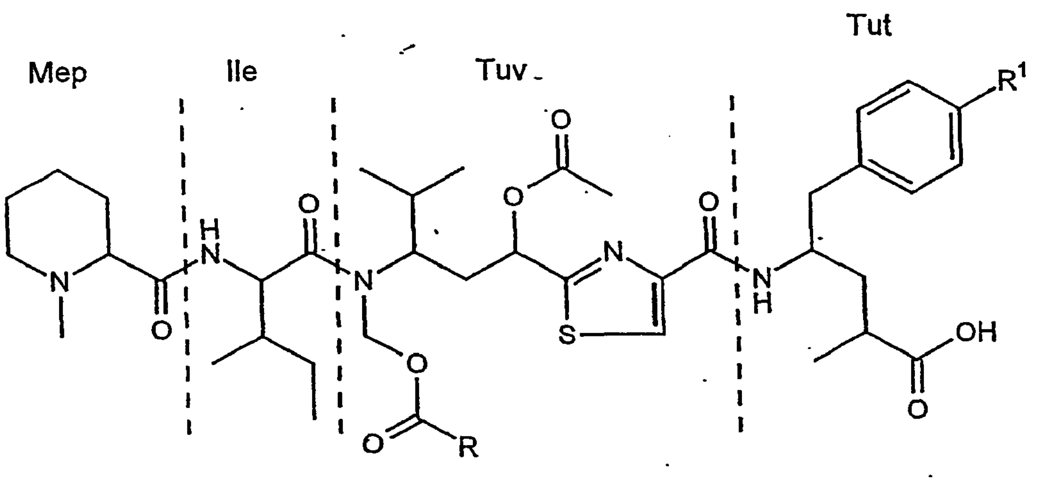

| | | R | $R^1$ |
|---|---|---|---|
| **1** | Tubulysin A | *i*-$C_4H_9$ | OH |
| **2** | Tubulysin B | $C_3H_7$ | OH |
| **3** | Tubulysin C | $C_2H_5$ | OH |
| **4** | Tubulysin D | *i*-$C_4H_9$ | H |
| **5** | Tubulysin E | $C_3H_7$ | H |
| **6** | Tubulysin F | $C_2H_5$ | H |

Aufgabe der Erfindung ist es, neue Tubulysine, Verfahren zu ihrer Herstellung und Mittel mit Tubulysinen bereitzustellen, insbesondere als Cytostatika.

Eine Ausführungsform der Erfindung betrifft eine Verbindung der folgenden allgemeinen Formel I (Tubulysin):

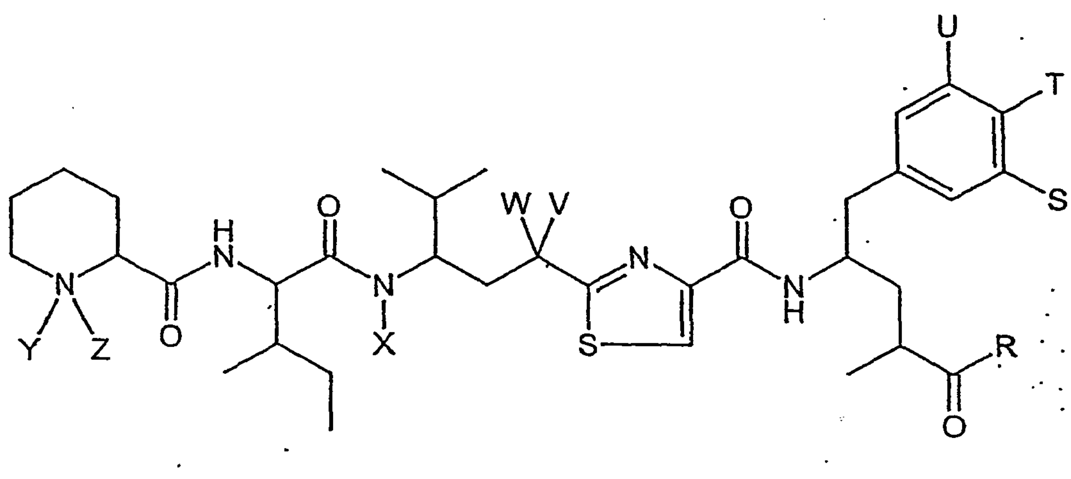

mit den folgenden Bedeutungen für R, $R^1$ $R^2$, $R^3$, $R^4$, $R^5$, $R^6$, $R^7$, $R^8$, $R^9$, $R^{10}$, $R^{11}$, S, T, U, V, W, X, Y und Z:

R = H, Alkyl, Aryl, $OR^1$, $NR^1R^2$ oder NH-$(CH_2)_{2-4}$-

$R^1$ = H, Alkyl oder Aryl
$R^2$ = H, Alkyl oder Aryl
S = H, Hal, $NO_2$ oder $NHR^3$
U = H, Hal, $NO_2$ oder $NHR^3$
$R^3$ = H, HCO oder $C_{1-4}$-Alkyl-CO
T = H oder $OR^4$
$R^4$ = H, Alkyl, Aryl, $COR^5$, P(O) $(OR^6)_2$ oder $SO_3R^6$
$R^5$ = Alkyl, Alkenyl, Aryl oder Heteroaryl
$R^6$ = H, Alkyl oder Metallion
V = H, $OR^7$, Hal oder (mit W) O
$R^7$ = H, Alkyl oder $COR^8$
$R^8$ = Alkyl, Alkenyl oder Aryl
W = H oder Alkyl oder (mit V) O
X = H, Alkyl, Alkenyl oder $CH_2OR^9$
$R^9$ = H, Alkyl, Alkenyl, Aryl oder $COR^{10}$
$R^{10}$ = Alkyl, Alkenyl, Aryl oder Heteroaryl
Y = (für Z = $CH_3$ oder $COR^{11}$) freies Elektronenpaar oder (für Z = $CH_3$) O
$R^{11}$ = Alkyl, $CF_3$ oder Aryl und/oder
Z = (für Y = 0 oder freies Elektronenpaar) $CH_3$ oder (für Y = freies Elektronenpaar) $COR^{11}$.

Bei Alkyl kann es sich um verzweigtes, unverzweigtes oder zyklisches $C_{1-20}$-Alkyl, insbesondere $C_{1-7}$-Alkyl, vorzugsweise $C_{1-6}$-Alkyl und besonders bevorzugt um $C_{1-4}$-Alkyl, insbesondere Methyl, Ethyl, Propyl, Isopropyl, n-Butyl, iso-Butyl, sek-Butyl, tert-Butyl, handeln. Cycloalkyl hat vorzugsweise 3 bis 8 C-Atome im Ring

Bei den Alkenylgruppen kann es sich um verzweigtes, unverzweigtes oder zyklisches $c_{2-20}$-Alkenyl, insbesondere $C_{2-7}$-Alkenyl, vorzugsweise $C_{2-6}$-Alkenyl und besonders bevorzugt um $C_{2-4}$-Alkenyl, insbesondere Vinyl, Allyl, Propen-1-yl, Propen-2-yl, But-1-en-1-yl, But-1-en-2-yl, But-1-en-3-yl, But-1-en-4-yl, But-2-en-1-yl, But-2-en-2-yl, 2-Methyl-propen-1-yl, 2-Methyl-propen-3-yl, handeln. Cycloalkenyl hat vorzugsweise 3 bis 8 C-Atome im Ring. Die Anzahl der Doppelbindungen der Alkenylgruppen kann 1 bis 3 betragen.

Aryl kann sein Phenyl, Naphthyl und Biphenylyl.

Heteroaryl kann sein Furyl, Thienyl, Imidazolyl, Indolyl, Pyridinyl, Pyrrolyl, Thiazolyl, Oxazolyl und Pyrimidinyl.

Alkyl, Alkenyl, Aryl und Heteroaryl können unsubstituiert oder substituiert sein, so können sie in beliebiger Position 1 bis 3 Substituenten aus der durch $C_{1-3}$-Alkyl, $C_{1-3}$-Alkoxy, Hydroxy, Amino ($NE_2$) oder Nitro ($NO_2$) gebildeten Gruppe tragen.

So kann eine erfindungsgemäße Verbindung aufweisen:

R, $R^1$, $R^4$, $R^5$, $R^8$, $R^9$, $R^{10}$ und/oder $R^{11}$ = unsubstituiertes oder substituiertes Phenyl, insbesondere $C_{1-4}$-Alkyl-substituiertes Phenyl
$R^5$ = $C_{1-4}$-Alk-yl, $C_{2-6}$-Alkenyl oder Pyridyl
$R^5$ und/oder X = $C_{2-4}$-Alkenyl
$R^6$ = Alkalimetall-Ion, insbesondere Na-Ion, oder Erdalkalimetall-Ion
$R^8$ und/oder $R^9$ = $C_{2-4}$-Alkenyl und/oder
$R^{10}$ = $C_{2-6}$-Alkenyl, insbesondere $C_{2-4}$-Alkenyl, oder Pyridyl.

Eine weitere Ausführungsform der Erfindung (Schema 1) betrifft ein Verfahren zur Herstellung einer Verbindung der allgemeinen Formel I (Typ 7) mit R = $OR^1$, $R^1$ = H, S = U = H, T = H oder OH, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = H, Y = freies Elekronenpaar und Z = $CH_3$, bei dem man eine Verbindung der folgenden allgemeinen Formel II (Typ 1, 2, 3, 4, 5 oder 6):

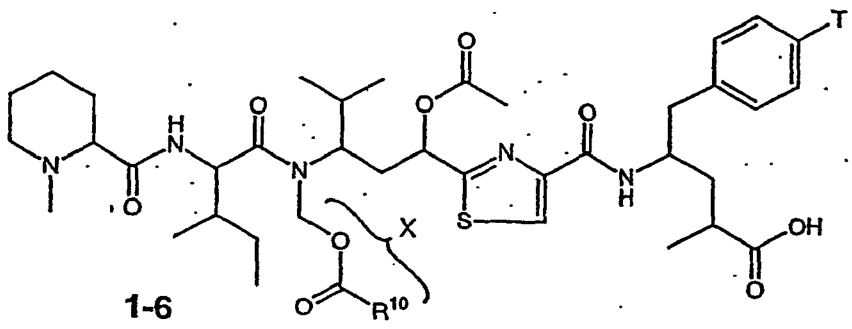

1-6

mit X = $CH_2OR^9$, $R^9$ = $COR^{10}$, $R^{10}$ = Alkyl und insbesondere $C_{1-6}$-Alkyl und im übrigen den vorstehend angegebenen Bedeutungen einer Esterspaltung in saurem Medium unterwirft und die Verbindung der allgemeinen Formel I mit den angegebenen Bedeutungen gewinnt.

Bei dem erfindungsgemäßen Verfahren kann man die Esterspaltung in einem organischen Lösungsmittel, insbesondere Dioxan, in Gegenwart einer Säure, insbesondere Chlorwassertoff, und/oder bei erhöhter Temperatur durchführen.

Eine weitere Ausführungsform der Erfindung (Schema 1) betrifft ein Verfahren zur Herstellung einer Verbindung der allgemeinen Formel I (Typ 8) mit R = $OR^1$, $R^1$ = H, S = U = H, T = H oder OH, V = $OR^7$, $R^7$ = $COR^8$, $R^8$ = Alkyl und insbesondere $C_{1-4}$-Alkyl, insbesondere Methyl, W = H, X = H, Y = freies Elektronenpaar und Z = $CH_3$, bei dem man eine Verbindung der allgemeinen Formel II (Typ 1, 2, 3, 4, 5 oder 6) mit X = $CH_2OR^9$, $R^9$ = $COR^{10}$, $R^{10}$ = Alkyl und insbesondere $C_{1-6}$-Alkyl, und im übrigen den vorstehend angegebenen Bedeutungen einer Acetal-Spaltung unterwirft und die Verbindung der allgemeinen Formel I mit den angegebenen Bedeutungen gewinnt.

Bei dem erfindungsgemäßen Verfahren kann man die Acetal-Spaltung in saurem Milieu, insbesondere in Gegenwart von Salzsäure, und/oder bei erhöhter Temperatur durchführen.

Eine weitere Ausführungsform der Erfindung (Schema 1) betrifft ein Verfahren zur Herstellung einer Verbindung der allgemeinen Formel I (Typ 9) mit R = $OR^1$, $R^1$ = H, S = U = H, T = H oder OH, V = $OR^7$, $R^7$ = H, W = H, X = $CH_2OR^9$ , $R^9$ = $COR^{10}$, $R^{10}$ = Alkyl und insbesondere $C_{1-6}$-Alkyl, Y = freies Elektronenpaar und Z = $CH_3$, bei dem man eine Verbindung der allgemeinen Formel II (Typ 1, 2, 3, 4, 5 oder 6) mit V = $OR^7$, $R^7$ = $COR^8$, $R^8$ = Alkyl, vorzugsweise $C_{1-4}$-Alkyl, insbesondere Methyl, und im übrigen den vorstehend angegebenen Bedeutungen einer Esterspaltung in schwach alkalischem Medium unterwirft und die Verbindung der allgemeinen Formel I mit den angegebenen Bedeutungen gewinnt.

Bei dem erfindungsgemäßen Verfahren kann man die Esterspaltung in einem organischen Medium, insbesondere einem hydrophilen organischen Lösungsmittel, vorzugsweise einem Alkohol, insbesondere Methanol, in Gegenwart einer schwachen Base durchführen, insbesondere $NH_3$.

Eine weitere Ausführungsform der Erfindung (Schema 1) betrifft ein Verfahren zur Herstellung einer Verbindung der allgemeinen Formel I (Typ 10) mit R = $OR^1$, $R^1$ = H, S = U = H, T = H oder OH, V = $OR^7$, $R^7$ = H, W = H, X = H, Y = freies Elektronenpaar und Z = $CH_3$, bei dem man eine Verbindung der allgemeinen Formel II (Typ 1, 2, 3, 4, 5 oder 6) mit V = $OR^7$, $R^7$ = $COR^8$, $R^8$ = Alkyl, vorzugsweise $C_{1-4}$-Alkyl, insbesondere Methyl, X = $CH_2OR^9$, $R^9$ = $COR^{10}$, $R^{10}$ = Alkyl und insbesondere $C_{1-6}$-Alkyl, und im übrigen den vorstehend angegebenen Bedeutungen einer doppelten Esterspaltung in stark alkalischem Medium unterwirft und die Verbindung der allgemeinen Formel I mit den angegebenen Bedeutungen gewinnt.

Bei dem erfindungsgemäßen Verfahren kann man die doppelte Esterspaltung in einem organischen Medium, insbesondere in einem hydrophilen organischen Lösungsmittel, vorzugsweise Alkohol, insbesondere Methanol, in Gegenwart einer starken Base durchführen, insbesondere eines Alkalimetallhydroxids, vorzugsweise von Natriumhydroxid.

Eine weitere Ausführungsform der Erfindung (Schema 1) betrifft ein Verfahren zur Herstellung einer Verbindung der folgenden allgemeinen Formel III (Typ 11):

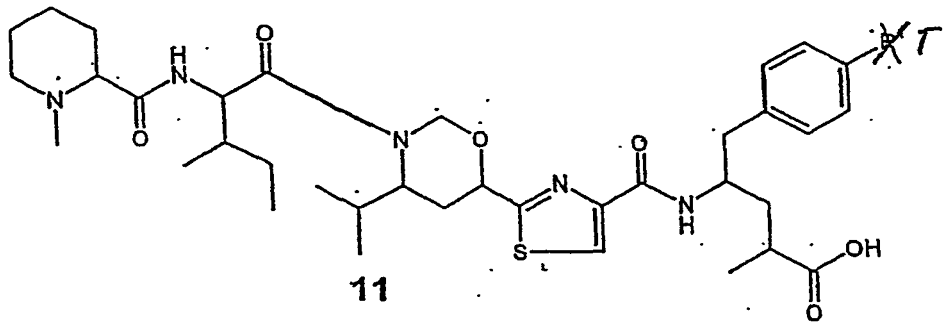

**11**

mit R = $OR^1$, $R^1$ = H, S = U = H, T = H oder $OR^4$, $R^4$ = H, V mit X = $CH_2O$-Brücke, W = H, Y = freies Elektronenpaar und Z = $CH_3$ in der allgemeinen Formel I, bei dem man eine Verbindung der allgemeinen Formel II (Typ 1, 2, 3, 4, 5 oder 6) mit X = $CH_2OR^9$, $R^9$ = $COR^{10}$, $R^{10}$ = Alkyl und insbesondere $C_{1-6}$-Alkyl, V = $OR^7$, $R^7$ = $COR^8$, $R^8$ = Alkyl, vorzugsweise $C_{1-4}$-Alkyl, insbesondere Methyl, und im übrigen den vorstehend angegebenen Bedeutungen einer Ringbildung unter doppelter Esterspaltung in saurem Medium unterwirft und die Verbindung der vorstehenden allgemeinen Formel mit den angegebenen Bedeutungen gewinnt.

Bei dem erfindungsgemäßen Verfahren kann man die Ringbildung in wässerigem Medium, in Gegenwart einer anorganischen Säure, vorzugsweise Salzsäure, und unter Erhitzen durchführen.

Eine weitere Ausführungsform (Schema 2) betrifft ein Verfahren zur Herstellung einer Verbindung der allgemeinen Formel I (Typ 12) mit R = $OR^1$, $R^1$ = H, S = U = H, T = H oder $OR^4$, $R^4$ = $COR^5$, $R^5$ = Alkyl und insbesondere $C_{1-6}$-Alkyl, Alkenyl und insbesondere $C_{2-6}$-Alkenyl, Aryl oder Heteroaryl, V = $OR^7$, $R^7$ = $COR^8$, $R^8$ = Alkyl, vorzugsweise $C_{1-4}$-Allyl, insbesondere Methyl, W = H, X = $CH_2OR^9$, $R^9$= $COR^{10}$, $R^{10}$ = $R^5$, Y = freies Elektronenpaar und Z = $CH_3$, bei dem man eine Verbindung der folgenden allgemeinen Formel IV (Typ 7):

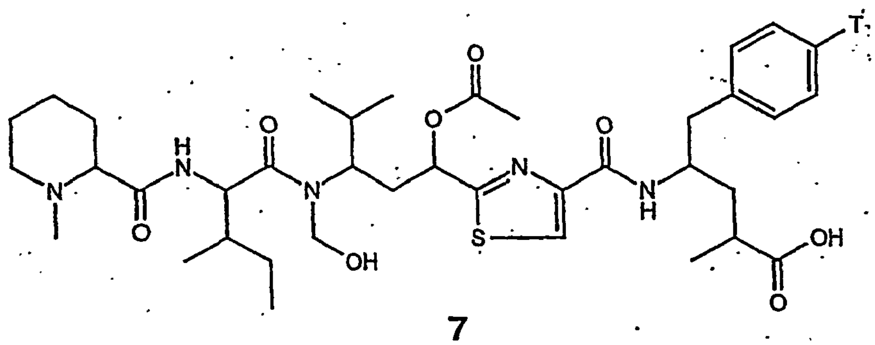

**7**

mit X = $CH_2OR^9$, $R^9$ = H und im übrigen den vorstehend angegebenen Bedeutungen einer Acylierung unterwirft und eine Verbindung der allgemeinen Formel I mit den angegebenen Bedeutungen gewinnt.

Bei dem erfindungsgemäßen Verfahren kann man mit einem Acylhalogenid, insbesondere Acylchlorid, und/ oder in Gegenwart einer schwachen Base acylieren, insbesondere einer schwachen organischen Base, vorzugsweise eines tertiären Amins, insbesondere Triethylamin.

Eine weitere Ausführungsform der Erfindung (Schema 2) betrifft ein Verfahren zur Herstellung einer Verbindung der allgemeinen Formel I (Typ 13) mit R = $OR^1$, $R^1$ = H, S = U = H, T = H oder $OR^4$, $R^4$ = H, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl und insbesondere $C_{1-6}$-Alkyl, Alkenyl und insbesondere $C_{2-6}$-Alkenyl, Aryl oder Heteroaryl, Y = freies Elektronenpaar und Z = $CH_3$, bei dem man bei einem erfindungsgemäßen Produkt mit T = $OR^4$, $R^4$ = $COR^5$ und $R^5$ = Alkyl und insbesondere $C_{1-6}$-Alkyl, Alkenyl und insbesondere $C_{2-6}$-Alkenyl, Aryl oder Heteroaryl und im übrigen den vorstehend angegebenen Bedeutungen in alkalischem Medium verseift und eine Verbindung der allgemeinen Formel I mit den angegebenen Bedeutungen gewinnt.

Bei dem erfindungsgemäßen Verfahren kann man mit Ammoniak verseifen.

Eine weitere Ausführungsform der Erfindung (Schema 3) betrifft ein Verfahren zur Herstellung einer Verbindung der allgemeinen Formel I (Typ 14) mit R = $DR^1$, $R^1$ = H, S = U = H, T = H oder OH, V = $OR^7$, $R^7$ = $COR^8$, $R^8$ = Alkyl,

vorzugsweise $C_{1-4}$-Alkyl, insbesondere Methyl, W = H, X = $CH_2OR^9$, $R^9$ = Alkyl und insbesondere $C_{1-4}$-Alkyl, Alkenyl und insbesondere $C_{2-6}$-Alkenyl, oder Aryl, Y = freies Elektronenpaar und Z = $CH_3$, bei dem man Verbindung der allgemeinen Formel II (Typ 1, 2, 3, 4, 5 oder 6) einer Esterspaltung unterwirft und alkyliert und eine Verbindung der allgemeinen Formel I mit den angegebenen Bedeutungen gewinnt.

Bei dem erfindungsgemäßen Verfahren kann man mit einem Alkylierungsmittel der Formel $R^9OH$ mit $R^9$ = Alkyl und insbesondere $C_{1-4}$-Alkyl, Alkenyl oder Aryl umsetzen.

Bei dem erfindungsgemäßem Verfahren kann man in Gegenwart von p-$CH_3$-$C_6H_4SO_2OH$ in Tetrahydrofuran (THF) bei erhöhter Temperatur umsetzen.

Eine weitere Ausführungsform der Erfindung (Schema 4) betrifft ein Verfahren zur Herstellung einer Verbindung der allgemeinen Formel I (Typ 15) mit R = $OR^1$, $R^1$ = H, S = U = H, T = H oder $OR^4$, $R^4$ = H, V = $OR^7$, $R^7$ = H oder $COR^8$, $R^8$ = Alkyl, vorzugsweise $C_{1-4}$-Alkyl, insbesondere Methyl, W = H, X = $CH_3$, Y = freies Elektronenpaar und Z = $CH_3$, bei dem man eine Verbindung der allgemeinen Formel IV (Typ 7) mit X = $CH_2OR^9$, $R^9$ = H und im übrigen den vorstehend angegebenen Bedeutungen einer Reduktion unterwirft und die Verbindung der allgemeinen Formel I mit den angegebenen Bedeutungen gewinnt.

Bei dem erfindungsgemäßen Verfahren kann man die Reduktion mit $NaCNBH_3$ und Trifluoressigsäure in Methanol (MeOH) durchführen.

Eine weitere Ausführungsform der Erfindung (Schema 4) betrifft ein Verfahren zur Herstellung einer Verbindung der allgemeinen Formel I (Typ 15) mit R = $OR^1$, $R^1$ = H, S = U = H, T = H oder $OR^4$, $R^4$ = H, V = $OR^7$, $R^7$ = H oder $COR^8$, $R^8$ = Alkyl, vorzugsweise $C_{1-4}$-Alkyl, insbesondere Methyl, W = H, X = $CH_3$, Y = freies Elektronenpaar und Z = $CH_3$, bei dem man eine Verbindung der allgemeinen Formel III (Typ 11) einer Ringöffnung unter Reduktion bzw. Reduktion unter Ringöffnung unterwirft und eine Verbindung der allgemeinen Formel I mit den angegebenen Bedeutungen gewinnt.

Bei dem erfindungsgemäßen Verfahren kann man in Gegenwart von $NaCNBH_3$ und $Me_3SiCl$ in Acetonitril ($CH_3CN$) umsetzen.

Eine weitere Ausführungsform der Erfindung (Schema 5) betrifft ein Verfahren zur Herstellung einer Verbindung der allgemeinen Formel I (Typ 16) mit R = $OR^1$, $R^1$ = H, S = U = H, T = H oder OH, V = $OR^7$, $R^7$ = $COR^8$, $R^8$ = Alkyl und insbesondere $C_{1-4}$-Alkyl, Alkenyl oder Aryl, W = H, X = $CH_2OR^9$, $R^9$ = $COR^{10}$, $R^{10}$ = Alkyl und insbesondere $C_{1-6}$-Alkyl oder Alkenyl, Y = freies Elektronenpaar und Z = $CH_3$, bei dem man eine erfindungsgemäße Verbindung der allgemeinen Formel I (Typ 9) mit V = $OR^7$ und $R^7$ = H und im übrigen den vorstehend angegebenen Bedeutungen einer Acylierung unterwirft und die Verbindung der allgemeinen Formel I mit den angegebenen Bedeutungen gewinnt.

Bei dem erfindungsgemäßen Verfahren kann man die Acylierung mit einem Acylhalogenid der Formel $R^8COCl$ mit $R^8$ = Alkyl und insbesondere $C_{1-4}$-Alkyl, Alkenyl oder Aryl, insbesondere Acylchlorid, und/oder in Gegenwart einer Base durchführen, insbesondere einer organischen Base, vorzugsweise eines Trialkylamins, insbesondere Triethylamin.

Eine weitere Ausführungsform der Erfindung (Schema 5) betrifft ein Verfahren zur Herstellung einer Verbindung der allgemeinen Formel I (Typ 17) mit R = $OR^1$, $R^1$ = H, S = U = H, T = H oder $OR^4$, $R^4$ = H, V = H oder F, W = H, X = $CH_2OR^9$, $R^9$ = $COR^{10}$, $R^{10}$ = Alkyl und insbesondere $C_{1-6}$-Alkyl oder Alkenyl, Y = freies Elektronenpaar und Z = $CH_3$, bei dem man eine erfindungsgemäße Verbindung der allgemeinen Formel I (Typ 9) mit V = $OR^7$ und $R^7$ = H und im übrigen den vorstehend angegebenen Bedeutungen einer katalytischen Hydrierung oder einer Fluorierung unterwirft und die Verbindung der allgemeinen Formel I mit den angegebenen Bedeutungen gewinnt.

Bei dem erfindungsgemäßen Verfahren kann man für V = H die Hydrierung mit Palladium/Kohlenstoff in Gegenwart von Essigsäure oder für V = F die Fluorierung mit DAST in Tetrahydrofuran durchführen.

Eine weitere Ausführungsform der Erfindung (Schema 5) betrifft ein Verfahren zur Herstellung einer Verbindung der allgemeinen Formel I (Typ 18) mit R = $OR^1$, $R^1$ = H, S = U = H, T = H oder $OR^4$, $R^4$ = H, V mit W = O, X = $CH_2OR^9$, $R^9$ = $COR^{10}$, $R^{10}$ = Alkyl und insbesondere $C_{1-6}$-Alkyl oder Alkenyl, Y = freies Elektronenpaar und Z = $CH_3$, bei dem man eine erfindungsgemäße Verbindung der allgemeinen Formel I (Typ 9) mit V = $OR^7$ und $R^7$ = H und im übrigen den vorstehend angegebenen Bedeutungen einer Oxidation unter Bildung eines Ketons unterwirft und eine Verbindung der allgemeinen Formel I mit den angegebenen Bedeutungen gewinnt.

Bei dem erfindungsgemäßen Verfahren kann man die Oxidation in Gegenwart von TPAP und NMO in Dichlormethan durchführen.

Eine weitere Ausführungsform der Erfindung (Schema 5) betrifft ein Verfahren zur Herstellung einer Verbindung der allgemeinen Formel I (Typ 19) mit R = $OR^1$, $R^1$ = H, S = U = H, T = H oder OH, V = $OR^7$, $R^7$ = H, W = Alkyl und insbesondere $C_{1-4}$-Alkyl, X = $CH_2OR^9$, $R^9$ = $COR^{10}$, $R^{10}$ = Alkyl und insbesondere $C_{1-6}$-Alkyl oder Alkenyl, Y = freies Elektronenpaar und Z = $CH_3$, bei dem man ein Produkt des vorstehenden erfindungsgemäßen Verfahrens (Typ 18) mit einer Grignard-Verbindung zur Verbindung der allgemeinen Formel I mit den angegebenen Bedeutungen umsetzt.

Bei dem erfindungsgemäßen Verfahren kann man die Umsetzung mit einer magnesiumorganischen Verbindung der Formel WMgHal mit W = Alkyl und insbesondere $C_{1-4}$-Alkyl durchführen.

Eine weitere Ausführungsform der Erfindung (Schema 5) betrifft ein Verfahren zur Herstellung einer Verbindung der allgemeinen Formel I (Typ 19) mit R = $OR^1$, $R^1$ = H, S = U = H, T = H oder OH, V = $OR^7$, $R^7$ = H, W = Alkyl und insbesondere $C_{1-4}$-Alkyl, X = $CH_2OR^9$, $R^9$ = $COR^{10}$, $R^{10}$ = Alkyl und insbesondere $C_{1-6}$-Alkyl oder Alkenyl, Y = freies

Elektronenpaar und Z = $CH_3$, bei dem man

(i) in einer ersten Stufe ein erfindungsgemäßes Verfahren durchführt und eine erfindungsgemäße Verbindung (Typ 18) gewinnt und danach
(ii) in einer zweiten Stufe die angefallene erfindungsgemäße Verbindung (Typ 18) in einem weiteren erfindungsgemäßen Verfahren zu einer Verbindung der allgemeinen Formel I mit den angegebenen Bedeutungen umsetzt und diese Verbindung gewinnt.

Eine weitere Ausführungsform der Erfindung (Schema 6) betrifft ein Verfahren zur Herstellung einer Verbindung der allgemeinen Formel I (Typ 20) mit R = $OR^1$, $R^1$ = Alkyl und insbesondere $C_{1-4}$-Alkyl oder Alkenyl, S = U = H, T = H oder $OR^4$, $R^4$ = H, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl und insbesondere $C_{1-6}$-Alkyl, Alkenyl und insbesondere $C_{2-6}$-Alkenyl, Aryl oder Heteroaryl, Y = freies Elektronenpaar und Z = $CH_3$, bei dem man eine Verbindung der allgemeinen Formel II (Typ 1, 2, 3, 4, 5 oder 6) oder ein Produkt eines erfindungsgemäßen Verfahrens (Typ 13) einer Alkylierung oder Alkenylierung unterwirft und eine Verbindung der allgemeinen Formel I mit den angegebenen Bedeutungen gewinnt.

Bei dem erfindungsgemäßen Verfahren kann man die Alkylierung oder Alkenylierung in Gegenwart von EDC, $R^1OH$ mit $R^1$ = Alkyl und insbesondere $C_{1-4}$-Alkyl oder Alkenyl und DMAP in Methylenchlorid durchführen.

Eine weitere Ausführungsform der Erfindung (Schema 6) betrifft ein Verfahren zur Herstellung einer Verbindung der allgemeinen Formel I (Typ 21) mit R = $NHR^1$, NH-$NR^1R^2$, $NHOR^1$ oder $NH(CH_2)_{2-4}NR^1R^2$, $R^1$ und $R^2$ unabhängig voneinander = H, Alkyl und insbesondere $C_{1-6}$-Alkyl oder Aryl, S = U = H, T = H oder $OR^4$, $R^4$ = H, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl und insbesondere $C_{1-6}$-Alkyl, Alkenyl und insbesondere $C_{2-6}$-Alkenyl, Aryl oder Heteroaryl, Y = freies Elektronenpaar und Z = $CH_3$, bei dem man eine Verbindung der allgemeinen Formel II (Typ 1, 2, 3, 4, 5 oder 6) oder ein Produkt eines erfindungsgemäßen Verfahrens (Typ 13) mit einer Verbindung der Formel RH einer Aminierung unterwirft, wobei R die angegebenen Bedeutungen besitzt, und eine Verbindung der allgemeinen Formel I mit den angegebenen Bedeutungen gewinnt.

Bei dem erfindungsgemäßen Verfahren kann man die Umsetzung

(i) in Gegenwart von EDC in Methylenchlorid oder
(ii) in Gegenwart von i-Butylchlorformiat und Triethylamin in THF durchführen.

Eine weitere Ausführungsform der Erfindung (Schema 6) betrifft ein Verfahren zur Herstellung einer Verbindung der allgemeinen Formel I (Typ 22) mit R = Alkyl und insbesondere $C_{1-4}$-Alkyl oder Alkenyl, S = U = H, T = H oder $OR^4$, $R^4$ = H, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl und insbesondere $C_{1-6}$-Alkyl, Alkenyl und insbesondere $C_{2-6}$-Alkenyl, Aryl oder Heteroaryl, Y = freies Elektronenpaar und Z = $CH_3$, bei dem man eine Verbindung der allgemeinen Formel II (Typ 1, 2, 3, 4, 5 oder 6) oder ein Produkt eines erfindungsgemäßen Verfahrens (Typ 13) mit einer lithiumorganischen Verbindung der Formel RLi mit der angegebenen Bedeutung für R zu der Verbindung der allgemeinen Formel I mit den angegebenen Bedeutungen umsetzt.

Eine weitere Ausführungsform der Erfindung (Schema 6) betrifft ein Verfahren zur Herstellung einer Verbindung der allgemeinen Formel I (Typ 23) mit R = Aminorest von 1-(2-Amino-$C_{2-4}$-alkyl)-pyrrol-2,5-dion, S = U = H, T = H oder $OR^4$,$R^4$ = H, V = $OR^7$, $R^7$ = COR8, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl und insbesondere $C_{1-6}$-Alkyl, Alkenyl und insbesondere $C_{2-6}$-Alkenyl, Aryl oder Heteroaryl, Y = freies Elektronenpaar und Z = CH3, bei dem man eine Verbindung der allgemeinen Formel II (Typ 1, 2, 3, 4, 5 oder 6) oder ein Produkt eines erfindungsgemäßen Verfahrens (Typ 13) einer Aminierung mit 1-(2-Amino-$C_{2-4}$-alkyl)-pyrrol-2,5-dion unterwirft und die Verbindung der allgemeinen Formel I mit den angegebenen Bedeutungen gewinnt.

Bei dem erfindungsgemäßen Verfahren kann man die Aminierung in Gegenwart von EDC in Methylenchlorid durchführen.

Eine weitere Ausführungsform der Erfindung (Schema 7) betrifft ein Verfahren zur Herstellung einer Verbindung der allgemeinen Formel I (Typ 24) mit R = $OR^1$, $R^1$ = H, S = U = H, T = $OR^4$, $R^4$ = $P(O) (OR^6)_2$ mit $R^6$ = H oder Alkyl, insbesondere $C_{1-4}$-Alkyl, oder $R^4$ = $SO_3R^6$ mit $R^6$ = H, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl und insbesondere $C_{1-6}$-Alkyl, Alkenyl, insbesondere $C_{2-6}$-Alkenyl, Aryl oder Heteroaryl, Y = freies Elektronenpaar und Z = $CH_3$, bei dem man

(i) eine Verbindung der allgemeinen Formel II (Typ 1, 2 oder 3) oder
(ii) ein Produkt eines erfindungsgemäßen Verfahrens (Typ 13) mit

(a) einer Verbindung der Formel $P(O)(OR^6)_2OH$ mit $R^6$ = H oder Alkyl und insbesondere $C_{1-4}$-Alkyl oder
(b) $SO_3$

umsetzt und die Verbindung der allgemeinen Formel I mit den angegebenen Bedeutungen gewinnt.

Bei dem erfindungsgemäßen Verfahren kann man die Variante (a) in Gegenwart von $I_2$ und Pyridin in Methylenchlorid durchführen.

Bei dem erfindungsgemäßen Verfahren Verfahren kann man die Variante (b) mit Pyridin-$SO_3$ durchführen.

Eine weitere Ausführungsform der Erfindung (Schema 7) betrifft ein Verfahren zur Herstellung einer Verbindung der allgemeinen Formel I (Typ 25) mit R = $OR^1$, $R^1$ = H, S = U = H, T = $OR^4$, $R^4$ = $COR^5$, $R^5$ = Alkyl und insbesondere $C_{1-4}$-Alkyl, Alkenyl oder $N(R^{12})_2$, $R^{12}$ = Alkyl, V = $OR^7$, $R^7$ = $COR^8$, $R^8$ = Alkyl, vorzugsweise $C_{1-4}$-Alkyl, insbesondere Methyl, W = H, X = $CH_2O_R{}^9$, $R^9$ = $COR^{10}$, $R^{10}$ = Alkyl, insbesondere $C_{1-6}$-Alkyl, Alkenyl, insbesondere $C_{2-6}$-Alkenyl, Aryl oder Heteroaryl, bei dem man

(i) eine Verbindung der allgemeinen Formel II (Typ 1, 2 oder 3) oder
(ii) ein Produkt eines erfindungsgemäßen Verfahrens (Typ 13) einer Acylierung unterwirft und die Verbindung der allgemeinen Formel I mit den angegebenen Bedeutungen gewinnt.

Bei dem erfindungsgemäßen Verfahren kann man die Acylierung mit einem Acylhalogenid der Formel $R^5COCl$ mit $R^5$ = Alkyl und insbesondere $C_{1-4}$-Alkyl, Alkenyl oder $N(R^{12})_2$ und $R^{12}$ = Alkyl, insbesondere mit einem Acylchlorid, in Gegenwart einer organischen Base, insbesondere eines Trialkylamins, vorzugsweise Triethylamin, in einem organischen Lösungsmittel durchführen, insbesondere THF.

Eine weitere Ausführungsform der Erfindung (Schema 7) betrifft ein Verfahren zur Herstellung einer Verbindung der allgemeinen Formel I (Typ 26) mit R = $OR^1$, $R^1$ = Alkyl und insbesondere $C_{1-4}$-Alkyl oder Alkenyl, S = U = H, T = $OR^4$, $R^4$ = Alkyl und insbesondere $C_{1-4}$-Alkyl oder Alkenyl, V = $OR^7$, $R^7$ = $COR^8$, $R^8$ = Alkyl und insbesondere $C_{1-4}$-Alkyl, insbesondere Methyl, W = H, X = $CH_2OR^9$, $R^9$ = $COR^{10}$, $R^{10}$ = Alkyl und insbesondere $C_{1-6}$-Alkyl, Alkenyl, insbesondere $C_{2-6}$-Alkenyl, Aryl oder Heteroaryl, Y = freies Elektronenpaar und Z = $CH_3$, bei dem man

(i) eine Verbindung der allgemeinen Formel II (Typ 1, 2 oder 3) oder
(ii) ein Produkt eines erfindungsgemäßen Verfahrens (Typ 13) einer Alkylierung unterwirft und die Verbindung der allgemeinen Formel gemäß Anspruch 1 mit den angegebenen Bedeutungen gewinnt.

Bei dem erfindungsgemäßen Verfahren kann man mit einem Alkyliodid der Formel $R^4I$ mit $R^4$ = Alkyl und insbesondere $C_{1-4}$-Alkyl oder Alkenyl in Gegenwart einer schwachen Base, insbesondere $Ag_2O$, in einem organischen Lösungsmittel alkylieren, insbesondere Methylenchlorid.

Bei dem erfindungsgemäßen Verfahren kann man mit Diazomethan in einem organischen Lösungsmittel methylieren, insbesondere Methanol.

Eine weitere Ausführungsform der Erfindung (Schema 7)betrifft ein Verfahren zur Herstellung einer Verbindung der allgemeinen Formel I (Typ 27) mit R = $OR^1$, $R^1$ = H, S = U = H, T = $OR^4$, $R^4$ = Alkyl und insbesondere $C_{1-4}$-Alkyl oder Alkenyl, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl, insbesondere $C_{1-6}$-Alkyl, Alkenyl, insbesondere $C_{2-6}$-Alkenyl, Aryl oder Heteroaryl, Y = freies Elektronenpaar und Z = $CH_3$, bei dem man ein Produkt eines erfindungsgemäßen Verfahrens (Typ 26) enzymatisch einer partiellen Dealkylierung oder Dealkenylierung unterwirft und die Verbindung der allgemeinen Formel I mit den angegebenen Bedeutungen gewinnt.

Bei dem erfindungsgemäßen Verfahren kann man als Enzym eine Esterase verwenden, insbesondere Schweineleber-Esterase.

Eine weitere Ausführungsform der Erfindung (Schema 7) betrifft ein Verfahren zur Herstellung einer Verbindung der allgemeinen Formel I (Typ 27) R = $OR^1$, $R^1$ = H, S = U = H, T = $OR^4$, $R^4$ = Alkyl und insbesondere $C_{1-4}$-Alkyl oder Alkenyl, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = $C_{1-6}$-Alkyl, Alkenyl, insbesondere $C_{2-6}$-Alkenyl, Aryl oder Heteroaryl, bei dem man

(a) in einer ersten Stufe

(i) eine Verbindung der allgemeinen Formel II (Typ 1, 2 oder 3) oder
(ii) ein Produkt eines erfindungsgemäßen Verfahrens (Typ 13) einem erfindungsgemäßen Verfahren unterwirft und eine erfindungsgemäße Verbindung (Typ 26) gewinnt und

(b) in einer zweiten Stufe die angefallene erfindungsgemäße Verbindung (Typ 26) einem weiteren erfindungsgemäßen Verfahren zu einer Verbindung der allgemeinen Formel I mit den angegebenen Bedeutungen umsetzt und diese Verbindung gewinnt.

Eine weitere Ausführungsform der Erfindung (Schema 8) betrifft ein Verfahren zur Herstellung einer Verbindung

der allgemeinen Formel I (Typ 28 und ggf. 29) mit R = $OR^1$, $R^1$ = H, S = H oder Hal, T = $OR^4$, $R^4$ = H, U = Hal, V = $OR^7$, $R^7$ = $COR^8$, $R^8$ = Alkyl und insbesondere $C_{1-4}$-Alkyl, insbesondere Methyl, W = H, X = $CH_2OR^9$, $R^9$ = $COR^{10}$, $R^{10}$ = Alkyl und insbesondere $C_{1-6}$-Alkyl, Alkenyl, insbesondere $C_{2-6}$-Alkenyl, Aryl oder Heteroaryl, bei dem man

(i) eine Verbindung der allgemeinen Formel II (Typ 1, 2, 3, 4, 5 oder 6) oder
(ii) ein Produkt eines erfindungsgemäßen Verfahrens (Typ 13) einer Halogenierung oder Dihalogenierung in ortho-Stellung zum T-Substituenten unterwirft und die Verbindung der allgemeinen Formel I mit den angegebenen Bedeutungen gewinnt.

Bei dem erfindungsgemäßen Verfahren kann man die Halogenierung in Gegenwart von $C_5Cl_5NF$-triflat, $SO_2Cl_2$, NBS und ICl durchführen.

Eine weitere Ausführungsform der Erfindung (Schema 8) betrifft ein Verfahren zur Herstellung einer Verbindung der allgemeinen Formel I (Typ 30) mit R = $OR^1$, $R^1$ = H, S = H, T = $OR^4$, $R^4$ = H, U = $NO^2$, V = $OR^7$, $R^7$ = $COR^8$, $R^8$ = Alkyl, vorzugsweise $C_{1-4}$-Alkyl, insbesondere Methyl, W = H, X = $CH_2OR^9$ , $R^9$ = $COR^{10}$, $R^{10}$ = Alkyl, insbesondere $C_{1-6}$-Alkyl, Alkenyl, insbesondere $C_{2-6}$-Alkenyl, Aryl oder Heteroaryl, Y = freies Elektronenpaar und Z = $CH_3$, bei dem man

(i) eine Verbindung der allgemeinen Formel II (Typ 1, 2, 3, 4, 5 oder 6) oder
(ii) ein Produkt eines erfindungsgemäßen Verfahrens (Typ 13) einer Nitrierung in ortho-Stellung zum T-Substituenten unterwirft und die Verbindung der allgemeinen Formel I mit den angegebenen Bedeutungen gewinnt.

Bei dem erfindungsgemäßen Verfahren kann man die Nitrierung mit einem Alkalimetallnitrit, insbesondere Natriumnitrit, und Essigsäure in Gegenwart eines organischen Lösungsmittels durchführen, insbesondere Ethanol.

Eine weitere Ausführungsform der Erfindung (Schema 8) betrifft ein Verfahren zur Herstellung einer Verbindung der allgemeinen Formel I (Typ 31) mit R = $OR^1$, $R^1$ = H, S = H, T = $OR^4$, $R^4$ = H, U = $NH_2$, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl und insbesondere $C_{1-6}$-Alkyl, Alkenyl, insbesondere $C_{2-6}$-Alkenyl, Aryl oder Heteroaryl, Y = freies Elektronenpaar und Z = $CH_3$, bei dem man ein Produkt eines erfindungsgemäßen Verfahrens (Typ 30) einer katalytischen Reduktion unterwirft und die Verbindung der allgemeinen Formel I mit den angegebenen Bedeutungen gewinnt.

Bei dem erfindungsgemäßen Verfahren kann man mit elementarem Wasserstoff in Gegenwart von Palladium/Aktivkohle reduzieren, insbesondere in einem organischen Lösungsmittel, vorzugsweise Ethanol.

Eine weitere Ausführungsform der Erfindung (Schema 8) betrifft ein Verfahren zur Herstellung einer Verbindung der allgemeinen Formel I (Typ 31) mit R = $OR^1$, $R^1$ = H, S = H, T = $OR^4$, $R^4$ = H, U = $NH_2$, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl und insbesondere $C_{1-6}$-Alkyl, Alkenyl, insbesondere $C_{2-6}$-Alkenyl, Aryl oder Heteroaryl, Y = freies Elektronenpaar und Z = $CH_3$, bei dem man

(a) in einer ersten Stufe

(i) eine Verbindung der allgemeinen Formel II (Typ 1, 2, 3, 4, 5 oder 6) oder
(ii) ein Produkt eines erfindungsgemäßen Verfahrens (Typ 13) einem weiteren erfindungsgemäßen Verfahren unterwirft und eine erfindungsgemäße Verbindung (Typ 30) gewinnt und

(b) in einer zweiten Stufe das erhaltene Produkt (Typ 30) einem weiteren erfindungsgemäßen Verfahren unterwirft und die Verbindung der allgemeinen Formel I mit den angegebenen Bedeutungen gewinnt.

Eine weitere Ausführungsform der Erfindung (Schema 8) betrifft ein Verfahren zur Herstellung einer Verbindung der allgemeinen Formel I (Typ 32) mit R = $OR^1$, $R^1$ = H, S = H, T = $OR^4$, $R^4$ = H, U = $NHR^3$, $R^3$ = Alkyl-CO und insbesondere $C_{1-4}$-Alkyl-CO, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = = Alkyl und insbesondere $C_{1-6}$-Alkyl, Alkenyl, insbesondere $C_{2-6}$-Alkenyl, Aryl oder Heteroaryl, Y = freies Elektronenpaar und Z = $CH_3$, bei dem man ein Produkt eines erfindungsgemäßen Verfahrens (Typ 31) einer Alkylierung unterwirft und die Verbindung der allgemeinen Formel I mit den angegebenen Bedeutungen gewinnt.

Bei dem erfindungsgemäßen Verfahren kann man mit einem Säureanhydrid der Formel $(R^3)_2O$ mit $R^3$ = CO-$C_{1-4}$-Alkyl alkylieren.

Eine weitere Ausführungsform der Erfindung (Schema 8) betrifft ein Verfahren zur Herstellung einer Verbindung der allgemeinen Formel I (Typ 32) mit R = $OR^1$, $R^1$ = H, S = H, T = $OR^4$, $R^4$ = H, U = $NHR^3$, $R^3$ = Alkyl-CO und insbesondere $C_{1-4}$-Alkyl-CO, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl, vorzugsweise $C_{1-6}$-Alkyl, Alkenyl, insbesondere $C_{2-6}$-Alkenyl, Aryl oder Heteroaryl, bei dem man

(a) in einer fakultativen ersten Stufe

(i) eine Verbindung der allgemeinen Formel II (Typ 1, 2, 3, 4, 5 oder 6) oder
(ii) ein Produkt eines erfindungsgemäßen Verfahrens (Typ 13) einem weiteren erfindungsgemäßen Verfahrens unterwirft,

(b) in einer zweiten Stufe das erhaltene Produkt (Typ 30) einem weiteren erfindungsgemäßen Verfahren unterwirft und
(c) in einer dritten Stufe die angefallene erfindungsgemäße Verbindung (Typ 31) einem weiteren erfindungsgemäßen Verfahren unterwirft und

die Verbindung der allgemeinen Formel I mit den angegebenen Bedeutungen gewinnt.
Eine weitere Ausführungsform der Erfindung (Schema 9) betrifft ein Verfahren zur Herstellung einer Verbindung der allgemeinen Formel I (Typ 33) mit R = $OR^1$, $R^1$ = H, S = U = H, T = $OR^4$, $R^4$ = H, V = $OR^7$, $R^7$ = $COR^8$, $R^8$ = Alkyl, vorzugsweise $C_{1-4}$-Alky insbesondere Methyl, W = H, X = $CH_2OR^9$, $R^9$ = $COR^{10}$, $R^{10}$ = Alkyl und insbesondere $C_{1-6}$-Alkyl, Alkenyl, insbesondere $C_{2-6}$-Alkenyl, Aryl oder Heteroaryl, Y = 0 und Z = $CH_3$, bei dem man

(i) eine Verbindung der allgemeinen Formel II (Typ 1, 2, 3, 4, 5 oder 6) oder
(ii) ein Produkt eines erfindungsgemäßen Verfahrens (Typ 13) einer Reaktion zur Bildung eines N-Oxids unterwirft und die Verbindung der allgemeinen Formel I mit den angegebenen Bedeutungen gewinnt.

Bei dem erfindungsgemäßen Verfahren kann man die N-Oxid-Bildung mit m-CPBA in einem organischen Lösungsmittel durchführen, insbesondere Methylenchlorid.
Eine weitere Ausführungsform der Erfindung (Schema 9) betrifft ein Verfahren zur Herstellung einer Verbindung der allgemeinen Formel I (Typ 34) mit R = $OR^1$, $R^1$ = H, S = U = H, T = $OR^4$, $R^4$ = H, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl und insbesondere $C_{1-6}$-Alkyl, Alkenyl, insbesondere $C_{2-6}$-Alkenyl, Aryl oder Heteroaryl, Y = freies Elektronenpaar, Z = $COR^{11}$ und $R^{11}$ = Alkyl, vorzugsweise $C_{1-4}$-Alkyl, insbesondere Methyl, bei dem man das Produkt eines erfindungsgemäßen Verfahrens (Typ 33) mit einem Acylierungsmittel umsetzt und die Verbindung der allgemeinen Formel I mit den angegebenen Bedeutungen gewinnt.
Bei dem erfindungsgemäßen Verfahren kann man die Acylierung mit einem Säureanhydrid durchführen, insbesondere Essigsäureanhydrid, vorzugsweise bei erhöhter Temperatur.
Eine weitere Ausführungsform der Erfindung (Schema 9) betrifft ein Verfahren zur Herstellung einer Verbindung der allgemeinen Formel I (Typ 34) mit R = $OR^1$, $R^1$ = H, S = U = H, T = $OR^4$, $R^4$ = H, V = $OR^7$, $R^7$ = $COR^8$, $R^8$ - Alkyl, vorzugsweise $C_{1-4}$-Alkyl, insbesondere Methyl, W = H, X = $CH_2OR^9$, $R^9$ = $COR^{10}$, $R^{10}$ = Alkyl und insbesondere $C_{1-6}$-Alkyl, Alkenyl, insbesondere $C_{2-6}$-Alkenyl, Aryl oder Heteroaryl, Y = freies Elektronenpaar, Z = $COR^{11}$ und $R^{11}$ = Alkyl, vorzugsweise $C_{1-4}$-Alkyl, insbesondere Methyl, bei dem man

(a) in einer ersten Stufe

(i) eine Verbindung der allgemeinen Formel II (Typ 1, 2, 3, 4, 5 oder 6) oder
(ii) ein Produkt eines erfahrungsgemäßen Verfahrens (Typ 13) einem erfindungsgemäßen Verfahren unterwirft und

(b) in einer zweiten Stufe das erhaltene Produkt (Typ 33) einem weiteren erfindungsgemäßen Verfahren unterwirft und

die Verbindung der allgemeinen Formel gemäß Anspruch 1 mit den angegebenen Bedeutungen gewinnt.
Eine weitere Ausführungsform der Erfindung betrifft ein therapeutisches Mittel, insbesondere Cytostatikum, mit einer oder mehreren erfindungsgemäßen Verbindungen als Wirkstoff neben einem oder mehreren fakultativen üblichen Trägern und/oder einem oder mehreren fakultativen üblichen Verdünnungsmitteln.
Schließlich betrifft eine Ausführungsform der Erfindung ein therapeutisches Mittel, insbesondere Cytostatikum, mit einem oder mehreren Produkten eines erfindungsgemäßen Verfahrens als Wirkstoff neben einem oder mehreren fakultativen üblichen Trägern und/oder einem oder mehreren fakultativen üblichen Verdünnungsmitteln.
Nachstehend wird die Erfindung durch Beispiele näher erläutert.

## *Tubulysin-Derivat **7a**: $R^1$ = OH (Schema 1)*

9.9 mg (11.7 μmol) Tubulysin A (**1**) wurden in 200 μl Dioxan gelöst und mit 1 ml 0.1 M Salzsäure-Lösung versetzt. Der Reaktionsansatz wurde bei 50°C 8 h gerührt. Anschließend wurde die Mischung liophilisiert und der Rückstand mittels präp. HPLC ($CH_3CN/H_2O$ 35/65 mit 50 mM $NH_4Ac$, pH = 6.5), wobei 5.3 mg (59 %) **7a** erhalten wurden.

$\mathbf{R_f}$ 0.55; $[a]^{22}_D$ -7.0 (c 0.89 MeOH); **UV** (MeOH): $\lambda_{max}$ nm (lgε) 226 (4.13), 250 (3.91); **IR** (KBr): $\nu_{max}$ 3386, 2963, 2934, 1655, 1546, 1232 $cm^{-1}$; **$^1$H NMR** (DMSO-$D_6$ 600 MHz) : wie Tubulysin A (**1**) außer Tuv δ 8.06 (1H, s, H-3), 6.18 (1H, d, J = 11.9 Hz, H-11b), 5.37 (1H, d, J = 11.8 Hz, H-11a), 4.63 (1H, br, H-5), 4.10 (1H, br, H-7), 2.20 (1H, m, H-6b), 1.99 (1H, m, H-8), 1.98 (1H, m, H-6a), 1.91 (1H, m, H-2'b), 1.48 (1H, m, H-3'b), 1.44 (1H, m, H-3'a), 1.42 (1H, m, H-2'a), 0.92 (3H, d, J = 6.4 Hz, H-9), 0.80 (3H, t, J = 7.3 Hz, H-4'), 0.73 (3H, d, J = 6.2 Hz, H-10); **$^{13}$C NMR** (DMSO-$D_6$ 150 MHz): wie Tubulysin A (**1**), außer Tuv δ 178.0 (s, C-4), 174.4 (s, C-1'), 160.0 (s, C-1), 149.6 (s, C-2), 123.0 (d, C-3), 68.0 (t, C-11), 67.5 (d, C-5), 55.0 (d, C-7), 37.4 (t, C-2'), 35.7 (t, C-6), 30.6 (d, C-8), 20.1 (q, C-9), 19.5 (q, C-10), 17.7 (t, C-3'), 13.3 (q, C-4'); **DCI MS:** m/z $[M+H^+]$ 760 (4); **HRMS (DCI):** $C_{38}H_{58}M_5O_9S$: 760.3917 $[M+H]^+$ (ber.: 760.3955).

## *Tubulysin-Derivat **8a**: $R^1$ = OH (Schema 1)*

20.0 mg (23.7 μmol) Tubuylsin A (**1**) wurden mit 500 μl 0.1 M Salzsäure versetzt. Der Reaktionsansatz wurde 5 Minuten bei 100°C gerührt, anschließend abgekühlt und mit gesättigter $NaHCO_3$-Lösung neutralisiert (pH = 7). Nachdem dreimal mit Ethylacetat extrahiert wurde, wurden die vereinigten organischen Phasen eingeengt. Das Rohprodukt wurde mittels präp. HPLC ($CH_3CN/H_2O$ 35/65 mit 50 mM $NH_4Ac$, pH = 6.5) gereinigt, wobei 6.4 mg (37 %) **8a,** 2.7 mg (15 %) **7a** und 5.1 mg (31 %) **10a** erhalten wurden.

**8a:**

$\mathbf{R_f}$ 0.55 ; $[\alpha]^{22}_D$ -10.2 (c 1.0 MeOH); **UV** (MeOH): $\lambda_{max}$ nm (1gε) 225 (4.10), 250 (3.94); **IR** (KBr) $\nu_{max}$ 3389, 3251, 2962, 2934, 1658, 1547, 1228 $cm^{-1}$; **$^1$H NMR** (DMSO-$D_6$ 600 MHz) : wie Tubulysin A (**1**) außer Tuv δ 8.17 (1H, s, H-3), 7.92 (1H, br, NH-7), 5.76 (1H, dd, J = 10.5, 3.0 Hz, H-5), 3.86 (1H, m, H-7), 2.13 (1H, m, H-6b), 2.09 (3H, s, H-5OAc), 1.95 (1H, m, H-6a), 1.73 (1H, m, H-8), 0.84 (3H, d, J = 6.4 Hz, H-10), 0.83 (3H, d, J = 6.0 Hz, H-9), Ile δ 7.54 (1H, d, J = 9.3 Hz, NH-2), 4.18 (1H, dd, J = 9.1 Hz, H-2), 1.75 (1H, m, H-3), 1.48 (1H, m, H-4b), 1.07 (1H, m, H-4a), 0.85 (3H, m, H-6), 0.81 (3H, m, H-5); **$^{13}$C NMR** (DMSO-$D_6$ 150 MHz): wie Tubulysin A (1) außer Tuv δ 169.6 (s, C-50Ac), 169.6 (s, C-4), 159.8 (s, C-1), 149.8 (s, C-2), 124.0 (d, C-3), 69.5 (d, C-5), 49.5 (d, C-7), 36.4 (t, C-6), 31.7 (d, C-8), 20.6 (q, C-5OAc), 18.9 (q, C-9), 18.0 (q, C-10), Ile δ 171.1 (s, C-1), 56.7 (d, C-2), 36.2 (d, C-3), 24.3 (t, C-4), 15.6 (q, C-6), 10.6 (q, C-5) ; **DCI MS** m/z $[M+H^+]$ 730 (100), 672 (15) ; **HRMS (DCI)**: $C_{37}H_{56}N_5O_8S$: 730.3839 $[M+H]^+$ (ber.: 730.3850).

## *Tubulysin Derivat **9a**: R = i-$C_4H_9$, $R^1$ = OH (Schema 1)*

9.6 mg (11.4 μmol) Tubulysin A (**1**) wurden in 1 ml Methanol gelöst und in Abständen von drei Stunden mit jeweils 10 μl (133.6 μmol) 25 % Ammoniak versetzt und bei Raumtemperatur gerührt. Anschließend wurde der Reaktionsansatz mit 18 % Salzsäure auf pH 5 eingestellt und dreimal mit Ethylacetat extrahiert. Die vereinigten organischen Phasen wurden eingeengt und mittels PSC ($CH_2Cl_2$/MeOH 85/15) gereinigt. Es wurden 2.5 mg (27 %) **9a**, 2.3 mg (29 %) **10a** und 1.7 mg (18 %) **1** isoliert.

**9a:**

**ESI MS** (1 eV): 802 $[M+H]^+$; **$^1$H-NMR** (DMSO-$D_6$, 600 MHz): δ = 4.63 (br, 1H, H-5), 4.10 (br, 1H, H-7), 2.20 (m, 1H, H-6b), 1.99 (m, 1H, H-8), 1.98 (m, 1H, H-6a)

## *Tubulysin-Derivat **10a**: $R^1$ = OH (Schema 1)*

5.4 mg (6.8 μmol) Tubulysin A (**1**) wurden in 300 μl Methanol gelöst, mit 67.0 μl 1 M Natronlauge (67.6 μmol) versetzt und 15 min bei Raumtemperatur gerührt. Anschließend wurde der Reaktionsansatz mit Wasser verdünnt und mit 1 M Salzsäurelösung auf pH 7 eingestellt. Nach dreimaliger Extraktion mit Ethylacetat wurden die vereinigten organischen Phasen eingeengt. Der Rückstand wurde mittels präp. HPLC ($CH_3CN/H_2O$ 35/65 mit 50 mM $NH_4Ac$, pH = 6.5) gereinigt, wobei 2.5 mg (57 %) **10a** erhalten wurden.

**$R_f$** 0.40; $[\alpha]^{22}_D$ -3.0 (c 0.66 MeOH); **UV** (MeOH): $\lambda_{max}$ nm (1g ε) 225 (4.12), 250 (3.92); **IR** (KBr): $\nu_{max}$ 3376 $cm^{-1}$, 3285, 2960, 2929, 1656, 1547; **$^1$H NMR** (DMSO-$D_6$ 600 MHz): wie Tubulysin A (**1**) außer Tuv δ 8.06 (1H, s, H-3), 7.67 (1H, br, NH-7), 4.65 (1H, ddbr, J = 8.7 Hz, H-5), 3.97 (1H, m, H-7), 1.98 (1H, m, H-6b), 1.79 (1H, m, H-6a), 1.74 (1H, m, H-8), 0.85 (3H, d, J = 6.7 Hz, H-9), 0.84 (3H, d, J = 6.2 Hz, H-10), Ile δ 7.75 (1H, br, NH-2), 4.15 (1H, dd, J = 8.6 Hz, H-2), 1.81 (1H, m, H-3), 1.55 (1H, m, H-4b), 1.11 (1H, m, H-4a), 0.86 (3H, d, J = 6.4 Hz, H-6), 0.80 (3H, t, J = 7.2 Hz, H-5); **$^{13}$C NMR** (DMSO-$D_6$ 150 MHz): wie Tubulysin A (1) außer Tuv δ 177.9 (s, C-4), 160.1 (s, C-1), 149.7 (s, C-2), 122.8 (d, C-3), 67.9 (d, C-5), 50.3 (d, C-7), 40.5 (t, C-6), 31.8 (d, C-8), 19.0 (q, C-9), 18.1 (q, C-10), Ile δ 171.4 (s, C-1), 57.1 (d, C-2), 36.2 (d, C-3), 24.6 (t, C-4), 15.6 (q, C-6), 10.4 (q, C-5); **DCI MS:** m/z $[M+H^+]$ 688(100), 256 (12), 223 (6), 98 (4); **HRMS (DCI):** $C_{35}H_{54}N_5O_7S$: 688.3799 $[M+H]^+$ (ber.: 688.3744).

*Methylester von Tubulysin-Derivat **10a:***

2.5 mg (3.6 μmol) 10a wurden in 200 μl Methanol gelöst, mit etherischer Diazomethanlösung versetzt und 10 min bei Raumtemperatur gerührt. Die Reinigung erfolgte direkt über eine PSC ($CH_2Cl_2$/MeOH 85/15) und ergab 2.8 mg (62 %) Methylester von **10a.**

**$R_f$** ($CH_2Cl_2$/MeOH 85/15) : 0.32; **IR** (KBr): $\tilde{\nu}$ = 3380 $cm^{-1}$ (m), 2960 (s), 2931 (s), 2872 (w), 1743 (s), 1659 (vs), 1516 (m), 1371 (w), 1229 (s), 1092 (w); **UV** (MeOH): $\lambda_{max}$ (lg ε) = 204 nm (4.53), 226 (4.27), 244 (sh, 4.01), 276 (sh, 3.38); **DCI MS** (120 eV, $NH_3$): 702 $[M+H]^+$; **$^1$H-NMR** ($CD_3OD$, 300 MHz): δ = 1.18 (d, 3 H, Tut10-H), 3.64 (s, 3 H, Tut11-H).

## *Cyclo-Tubulysin A (11a): $R^1$ = OH (Schema 1)*

9.6 mg (11.3 μmol) Tubulysin A (1), verteilt als dünner Film an den Glaswandungen des Reaktions-gefäßes wurde mit 1 ml 0.5 M Salzsäure-Lösung versetzt und 30 min bei 100°C gerührt. Der Reaktionsansatz wurde anschließend lyophilisiert und der Rückstand über PSC ($CH_2Cl_2$/MeOH 90/10) gereinigt, wobei 3.9 mg (50 %) **11a** und 1.6 mg (21 %) **10a** erhalten wurden.

**11a:**

**ESI MS** (1 eV): 699 $[M+H]^+$; **$^1$H-NMR** (DMSO-$D_6$, 300 MHz): δ = 8.15 (s, 1 H, Tuvt3-H), 5.32 (d, 1 H, Tuv5-H), 1.7 (m, 1 H, Tuv6a-H), 2.3 (m, 1 H, Tuv6b-H), 4.32 (m, 1 H, Tuv7-H), 1.8 (m, 1 H, Tuv8-H), 0.75 (d, 3 H, Tuv9-$H_3$), 0.95 (d, 3 H, Tuv10-$H_3$), 4.86 (d, 1 H, Tuv11a-H), 5.65 (d, 1 H, Tuv11b-H).

*Tubulysin-Derivate **12 und 13***

Man kann aus einem Tubulysin-Derivat 7a ein Tubulysin-Derivat 12 herstellen, indem man 7a mit Acetylchlorid in Triethylamin umsetzt.

Geht man von einem Tubulysin-Derivat Typ 7 mit T = $OR^4$, $R^4$ = $COR^5$ und $R^5$ = Methyl oder Ethyl aus, so kann man das anfallende Tybulysin-Derivat Typ 12 mit Ammoniak zu einem Tubulysin-Derivat Typ 13 verseifen.

## *Tubulysin A-methylether (14a): $R^1$ = OH (Schema 3)*

Zu einer Lösung von 10.0 mg (11.9 μmol) Tubulysin A (1) in 500 μl abs. THF wurden 500 μl abs. Ethanol und 1 mg (5.3 μmol) *p*-Toluolsulfonsäure zugegeben. Der Reaktionsansatz wurde 20 Minuten bei 80°C gerührt. Anschließend wurde vom Lösungsmittel befreit und das Rohprodukt mittels PSC ($CH_2Cl_2$/MeOH 90/10) gereinigt. Es wurden 3.1 mg (33 %) **14a** erhalten.

**ESI MS** (1 eV): 788 $[M+H]^+$

*Tubulysin-Derivat **15***

Man kann ein Tubulysin-Derivat 15 dadurch herstellen, daß man ein Tubulysin-Derivat 7a mit $NaCNBH_3$ und TFA in Methanol reduziert.

*Tubulysin-Derivat **16***

Man kann ein Tubulysin-Derivat 16 dadurch herstellen, daß man ein Tubulysin-Derivat 9a mit Acetylchlorid in Triethylamin acetyliert.

*Tubulysin-Derivat **17***

Man kann ein Tubulysin-Derivat 17 dadurch herstellen, daß man ein Tubulysin-Derivat 9a in Gegenwart von $CH_3COOH$ bzw. DAST katalytisch an einem Pd/C-Katalysator mit elementarem Wasserstoff hydriert.

*Tubulysin-Derivate **18 und 19***

Man kann ein Tubulysin-Dervat 18 dadurch herstellen, daß man ein Tybulysin-Derivat 9a in Gegenwart von TPAP und NMO oxidiert.

Das erhaltene Tubulysin-Derivat 18 kann man mit Ethylmagnesiumbromid zu einem Tubulysin-Derivat 19 umsetzen.

## *Tubulysin A-methylester (**20a**): $R^2$ = $CH_3$ (Schema 6)*

19.0 mg (22.5 μmol)Tubulysin A (**1**) wurden in 300 μl Methanol gelöst und bei Raumtemperatur zweimal in Abständen von 15 Minuten mit etherischer Diazomethanlösung versetzt. Der Reaktionsansatz wurde eingeengt, die Reinigung des Rohproduktes erfolgte über PSC ($CH_2Cl_2$/MeOH 90/10) wobei 11.7 mg (61 %) **20a** erhalten wurden.

**IR** (KBr): $\tilde{\nu}$= 3383 $cm^{-1}$ (m), 2962 (s), 2875 (w), 1739 (vs), 1666 (vs), 1516 (s), 1227 (vs), 1091 (w); **UV** (MeOH): $\lambda_{max}$ (lg ε) = 203 nm (4.61), 225 (4.34), 243 (sh, 4.11), 276 (sh, 3.41); **DCI MS** (120 eV, *l*-Butan): 858 $[M+H]^+$; **$^1$H-NMR** (DMSO-$D_6$, 300 MHz): δ = 2.43 (m, 1 H, Tut2-H), 1.06 (d, 3 H, Tut10-H), 3.53 (s, 3 H, Tut11-H); **$^{13}$C-NMR** (DMSO-$D_6$, 75 MHz): δ = 175.8 (TutC1), 35.8 (TutC2), 17.6 (TutC10), 51.3 (TutC11).

## *Tubulysin-A-ethylester (**20b**): $R^1$ = OH, $R^2$ = $C_2H_5$ (Schema 6)*

Zu einer Lösung aus 5.2 mg (6.2 μmol) Tubulysin A (**1**) in 300 μl Dichlormethan wurden13.5 μl (9.3 μmol) Ethanol, 1.8 mg (9.3 μmol) EDC und 57 μl (9.3 μmol) einer DMAP-Lösung (5 mg/250 μl $CH_2Cl_2$) gegeben. Der Reaktionsansatz wurde bei Raumtemperatur über Nacht gerührt. Anschließend erfolgte eine Reinigung der Rohprodukte mittels PSC ($CH_2Cl_2$/MeOH 90/10), wobei 1.7 mg (32 %) **20b,** 0.7 mg (13 %) **25a** und 1.0 mg (18 %) **20b** mit $R^1$ = $OCOCH_3$ erhalten wurden.

**20b:**

**DCI MS** (120 eV, $NH_3$): 872 $[M+H]^+$; **HRMS (DCI)**: $C_{45}H_{69}N_5O_{10}S$: $[M+H]^+$ ber.: 872.4843 (gef.: 872.4917); **$^1$H-NMR** ($CD_3OD$, 300 MHz): δ = 2.58 (m, 1 H, Tut2-H), 1.19 (d, 3 H, Tut10-H), 4.11 (q, 2 H, Tut11-H), 1.23 (t, 3 H, Tut12-H).

**20b** mit $R^1$ = $OCOCH_3$

**$R_f$** ($CH_2Cl_2$/MeOH 90/10): 0.51; **IR** (KBr): $\tilde{\nu}$= 3392 $cm^{-1}$ (m), 2962 (s), 2920 (s), 2874 (w), 1736 (s), 1667 (vs), 1507 (m), 1370 (w), 1218 (s), 1195 (s); **UV** (MeOH): $\lambda_{max}$ (lg ε) = 203 nm (4.60), 218 (4.30), 227 (sh, 4.23), 248 (sh, 3.98); **DCI MS** (120 eV, $NH_3$): 914 $[M+H]^+$; **HRMS (DCI):** $C_{47}H_{71}N_5O_{11}S$: $[M+H]^+$ ber.: 814.4949 gef.: 914.5044; **$^1$H-NMR** ($CD_3OD$, 300 MHz): δ = 7.29 (d, 2 H, Tut7-H), 7.02 (d, 2 H, Tut8-H), 1.20 (d, 3 H, Tut10-H), 4.11 (q, 2 H, Tut11-H), 1.23 (t, 3 H, Tut12-H), 2.28 (s, 3-H, Tut13-H).

## *Tubulysin A-propylester (20c): $R^1$ = OH, $R^2$ = $C_3H_7$ (Schema 6)*

Eine Lösung aus 11.3 mg (13.4 µmol) Tubulysin A (**1**) in 450 µl Dichlormethan/Diethylether (1/2) wurde mit 6.5 µl (67.0 µmol) Propyliodid und 6.2 mg (26.8 µmol) Silber(I)oxid versetzt. Der Reaktionsansatz wurde über Nacht bei.Raumtemperatur gerührt, anschließend über Celite filtriert und der Rückstand mit Dichlormethan gewaschen. Die vereinigten organischen Phasen wurden eingeengt und mittels PSC ($CH_2Cl_2$/MeOH 90/10) gereinigt, wobei 7.6 mg (64 %) 20c erhalten wurden.

**IR** (KBr): $\tilde{\nu}$= 3387 $cm^{-1}$ (m), 2964 (s), 2938 (m), 2876 (w), 1737 (s), 1667 (vs), 1516 (s), 1416 (m), 1370 (w), 1225 (s), 1094 (w); **UV** (MeOH): $\lambda_{max}$ (lg ε) = 204 nm (4.64), 224 (4.39), 246 (sh, 4.12), 276 (3.60); **DCI MS** (120 eV, $NH_3$): 886 $[M+H]^+$; **$^1$H-NMR** ($CD_3OD$, 300 MHz): δ = 2.63 (m, 1 H, Tut2-H), 1.68 (m, 1 H, Tut3a-H), 2.03 (m, 1 H, Tut3b-H), 4.32 (m, 2 H, Tut4-H), 2.83 (m, 2 H, Tut5-H), 6.72 (d, 1 H, Tut7-H), 7.08 (d, 1 H, Tut8-H), 1.20 (d, 3 H, Tut10-H), 4.02 (t, 2 H, Tut11-H) 1.64 (m, 2 H, Tut12-H), 0.95 (t, 3 H, Tut13-H); **$^{13}$C-NMR** ($CD_3OD$, 75 MHz): δ = 177.9 (TutC1), 38.1 (TutC2), 38.9 (TutC3), 50.7 (TutC4), 41.5 (TutC5), 130.0 (TutC6), 116.2 (TutC7), 131.4 (TutC8), 157.1 (TutC9), 18.4 (TutC10), 67.2 (TutC11), 23.0 (TutC12).10.7 (TutC13).

## *Tubulysin-A-propylamid (**21a**): $R^1$ = OH, $R^2$ = $C_3H_7$ (Schema 6)*

Zu einer Lösung aus 4.9 mg (5.8 µmol) Tubulysin A (1) in 300 µl Dichlormethan wurden 180 µl (19.2 µmol) EDC-Lösung (4 mg/200 µl $CH_2Cl_2$) und 36 µl (43.5 µmol) Propylamin-Lösung (10 µl/100 µl $CH_2Cl_2$) gegeben. Der Reaktionsansatz wurde zwei Tage bei Raumtemperatur gerührt. Die Reinigung erfolgte mittels PSC ($CH_2Cl_2$/MeOH 90/10) und ergab 1.0 mg (20 %) **21a.**

**ESI MS** (1 eV): 885 $[M+H]^+$; **$^1$H-NMR** ($CD_3OD$, 300 MHz): δ = 2.49 (m, 1 H, Tut2-H), 1.14 (d, 3 H, Tut10-H), 3.15 (t, 2 H, Tut11-H), 1.56 (m, 2 H, Tut12-H), 0.96 (t, 3 H, Tut13-H).

## *Tubulysin-A-hexylamid (**21b**): $R^1$ = OH, $R^2$ = $C_6H_{13}$ (Schema 6)*

2.8 µl (16.5 µmol) Hünig-Base wurden in 200 µl abs. THF bei 0˚C gelöst und mit 1.4 µl (11.0 µmol) *i*-Butylchlorformiat versetzt. Nach 5 min wurden 9.3 mg (11.0 µmol) Tubulysin A (**1**), gelöst in 300 µl abs. THF, zugegeben und weitere 40 min bei 0˚C gerührt. Anschließend wurde der Reaktionsansatz mit 1.6 µl (12.1 µmol) Hexylamin und 2.8 µl (16.5 µmol) Hünig-Base versetzt und bei Raumtemperatur über Nacht gerührt. Eine Reinigung des Rohproduktes erfolgte direkt mittels PSC ($CH_2Cl_2$/MeOH 90/10) und lieferte neben 6.0 mg (65 %) **1**, 3.6 mg (35 %) **21b.**

**$R_f$** ($CH_2Cl_2$/MeOH 90/10): 0.41; **IR** (KBr): $\tilde{\nu}$ = 3389 $cm^{-1}$ (m), 2960 (s), 2932 (s), 2872 (w), 1743 (m), 1654 (vs), 1516 (m), 1418 (m), 1228 (s); **UV** (MeOH): $\lambda_{max}$ (1g ε) = 204 nm (4.62), 226 (4.31), 242 (sh, 4.09), 278 (sh, 3.41); **DCI MS** (120 eV, $NH_3$): 927 $[M+H]^+$; **HRMS (DCI):** $C_{49}H_{78}N_6O_9S$: $[M+H]^+$ ber.: 927.5629 (gef.: 927.5641).

## *Tubulysin-A-benzylamid (**21c**): $R^1$ = OH, $R^2$ = $CH_2C_6H_5$ (Schema 6)*

4.5 µl (26.8 µmol) Hünig-Base wurden in 200 µl abs. THF gelöst und auf 0˚C gekühlt. Die Lösung wurde mit 2.4 µl (17.9 µmol) Chlorameisensäureisobutylester versetzt und 5 min gerührt. Anschließend wurde eine Lösung von 10 mg (11.9 µmol) Tubulysin A in 300 µl abs. THF zugegeben und bei 0˚C gerührt. Nach 30 min wurden 1.4 µl (13.1 µmol) Benzylamin und 3 µl (17.9 µmol) Hünig-Base zugegeben und das Reaktionsgemisch über Nacht bei Raumtemperatur gerührt. Der Reaktionsansatz wurde direkt mittels PSC ($CH_2Cl_2$/Methanol 90/10) gereinigt, wobei 3.5 mg (37 %) **NT19** erhalten wurden.

**$R_f$** ($CH_2Cl_2$/MeOH 90/10): 0.40; **Drehwert:** $[\alpha]_D^{20} =$ + 27.2 (c 0.22, Methanol); **IR** (KBr): $\tilde{\nu}$= 3383 $cm^{-1}$ (m), 2962 (m), 2935 (m), 2875 (w), 1742 (m), 1661 (vs), 1516 (m), 1420 (w), 1371 (w), 1227 (s), 1094 (w) ; **UV** (MeOH): $\lambda_{max}$ (lg ε) = 204 nm (4.62), 224 (sh, 4.24), 246 (sh, 3.94), 277 (sh, 3.24); **DCI MS** (120 eV, $NH_3$): $[M+H]^+$; **ESI MS** (1 eV):

932 $[M+H]^+$; **$^{1}$H-NMR** ($CD_3OD$, 300 MHz): δ = 2.54 (m, 1 H, Tut2-H), 1.64 (m, 1 H, Tut3a-H), 2.03 (m, 1 H, Tut3b-H), 4.24 (m, 1 H, Tut4-H), 2.82 (bd, 2 H, Tut5-H), 7.01 (d, 2 H, Tut7-H), 6.69 (d, 2 H, Tut8-H), 1.17 (d, 3 H, Tut10-H), 4.42 (dd, 2 H, Tut11-H), 7.2-7.4 (m, 5 H, Tut13,14,15-H); **$^{13}$C-NMR** ($CD_3OD$, 75 MHz): δ = 187.5 (TutC1), 39.1 (TutC2), 40.3 (TutC3), 51.3 (TutC4), 41.3 (TutC5), 130.0 (TutC6), 131.4 (TutC7), 116.2 (TutC8), 157.0 (TutC9), 19.1 (TutC10), 44.2 (TutC11), 140.2 (TutC12), 128.7 (TutC13), 129.5 (TutC14), 128.1 (TutC15).

*Tubulysin-Derivat 22*

Ein Tubulysin-Derivat 22 kann man dadurch gewinnen, daß man Tubulysin 1 mit Methyl- oder Ethyllithium zum sekundären Amin reduziert.

*Tubulysin-Derivat **23***

Ein Tubulysin-Derivat 23 kamm man dadurch gewinnen, daß man Tubulysin 1 in Gegenwart von EDC in Methylenchlorid mit 1-(2-Aminoethyl)-pyrrol-2,5-dion amidiert.

*Tubulysin-Derivat **24***

Ein Tubulysin-Derivat 24 mit T = $OR^4$, $R^4 = SO_3R^6$ und $R^6$ = H kann man dadurch gewinnen, daß man Tubulysin 1 mit Pyridin-$SO_3$ umsetzt. Analog kann man Tubulysin 1 mit Phosphorsäuredimethylester in Gegenwart von Iod und Pyridin in Methylenchlorid umsetzen.

## *Acetyl-Tubulysin-A (25a): R = i-$C_4H_9$, $R^1$ = $CH_3$ (Schema 7)*

8.9 mg Tubulysin A (**1**) wurden in 200 µl abs. THF gelöst und mit 8.2 µl (30.6 µmol) Acetylchlorid und 7.1 µmol) Triethylamin versetzt. Der Reaktionsansatz wurde 15 min bei Raumtemperatur gerührt, anschließend mit 1 ml Wasser versetzt und dreimal mit Ethylacetat extrahiert. Die vereinigten organischen Phasen wurden eingeengt und am Hochvakuum getrocknet. Das Rohprodukt wurde mittels PSC ($CH_2Cl_2$/MeOH 90/10) gereinigt, wobei 5.6 mg (62 %) **25a** erhalten wurden.

**DCI MS** (120 eV, $NH_3$): 886 $[M+H]^+$; **HRMS (DCI)**: $C_{45}H_{67}N_5O_{11}S$: $[M+H]^+$ ber.: 886.4636 (gef.: 886.4701) ; **$^{1}$H-NMR** ($CD_3OD$, 300 MHz): δ = 2.58 (m, 1 H, Tut2-H), 1.73 (m, 1 H, Tut3a-H), 2.06 (m, 1 H, Tut3b-H), 4.39 (m, 1 H, Tut4-H), 2.98 (bd, 2 H, Tut5-H), 7.29 (d, 1 H, Tut7-H), 7.00 (d, 1 H, Tut8-H), 1.21 (d, 3 H, Tut10-H), 2.27 (s, 3 H, Tut12-H); **$^{13}$C-NMR** ($CD_3OD$, 75 MHz): δ = 181.1 (TutC1), 38.7 (TutC2), 39.4 (TutC3), 51.1 (TutC4), 41.2 (tut5), 137.2 (TutC6), 131.4 (TutC7), 122.5 (TutC8), 150.8 (TutC9), 18.8 (TutC10), 171.2 (TutC11), 20.9 (TutC12).

## *Isobutyryl-Tubulysin-A (25b): R = i-$C_4H_9$, $R^1$ = $CH(CH_3)_2$ (Schema 7)*

15.1 mg (17.8 µmol) Tubulysin A wurden in 400 µl abs. THF gelöst und mit 5.6 µl (53.4 µmol) i-Buttersäurechlorid und 12.5 µl (89.0 µmol) Triethylamin versetzt. Der Reaktionsansatz wurde 30 min bei Raumtemperatur gerührt, anschließend mit 2 ml Wasser versetzt und dreimal mit Ethylacetat extrahiert. Die vereinigten organischen Phasen wurden über Natriumsulfat getrocknet und eingeengt. Die Reinigung des Rohproduktes erfolgt mittels PSC ($CH_2Cl_2$/Methanol 90/10) und ergab 5.3 mg (32 %) **NT20**.

**$R_f$** ($CH_2Cl_2$/MeOH 90/10): 0.36; **Drehwert:** $[\alpha]_D^{20} =$ + 11.5 (c 0.35, Methanol); **IR** (KBr): $\tilde{\nu}$= 3392 $cm^{-1}$ (m), 2964 (m), 2936 (m), 2875 (w), 1755 (s), 1668 (vs), 1544 (w), 1508 (w), 1468 (w), 1420 (w), 1371 (w), 1227 (s), 1167 (w); **UV** (MeOH): $\lambda_{max}$ (lg ε) = 204 nm (4.54), 223 (sh, 4.20); **DCI MS** (120 eV, $NH_3$): $[M+H]^+$; **ESI MS** (1 eV): 913 $[M+H]^+$; **$^{1}$H-NMR** ($CD_3OD$, 300 MHz): δ = 2.59 (m, 1 H, Tut2-H), 1.73 (m, 1 H, Tut3a-H), 2.07 (m, 1 H, Tut3b-H), 4.39 (m, 1 H, Tut4-H), 2.98 (bd, 2 H, Tut5-H), 6.99 (d, 2 H, Tut7-H), 7.30 (d, 2 H, Tut8-H), 1.22 (d, 3 H, Tut10-H), 2.82 (d, 1 H, Tut12-H), 1.31 (d, 6 H, Tut13,14-H); **$^{13}$C-NMR** ($CD_3OD$, 75 MHz): δ = 181.1 (TutC1), 38.7 (TutC2), 39.4 (TutC3), 51.1 (TutC4), 41.2 (tut5), 137.2 (TutC6), 131.4 (TutC7), 122.3 (TutC8), 150.8 (TutC9), 18.8 (TutC10), 177.2 (TutC11),

35.3 (TutC12), 19.2 (TutC13,14).

## *Tubulysin A-allylether -allylester (26a): R = i-$C_4H_9$, $R^1$ = $CH_2CHCH_2$ (Schema 7)*

6.0 mg (7.1 μmol) Tubulysin A (1), gelöst in 300 μl Dichlormethan/Diethylether (1/1), wurden mit 6.2 μl (71.2 μmol) Allylbromid und 6.6 mg (28.5 μmol) Silber(I)oxid versetzt und über Nacht bei Raumtemperatur gerührt. Anschließend wurde über Celite filtriert. Der Rückstand wurde mit Dichlormethan gewaschen und die vereinigten organischen Phasen eingeengt. Die Reinigung des Rohproduktes erfolgte über PSC ($CH_2Cl_2$/MeOH 90/10) und liefert 2.9 mg (44 %) **26a.**

**ESI MS** (1 eV): 924 $[M+H]^+$; **$^1$H-NMR** ($CD_3OD$, 400 MHz): δ = 7.17 (d, 2 H, Tut7-H), 6.86 (d, 2 H, Tut8-H), 1.22 (d, 3 H, Tut10-H), 4.5-4.6 (m, 4 H, Tut11-H, Tut14-H), 5.93 (m, 1 H, Tut12-H), 5.1-5.5 (m, 4 H, Tut13-H, Tut16-H), 6.07 (m, 1 H, Tut15-H).

## *Tubulysin-A-methylether-methylester (26b): R = i-$C_4H_9$, $R^1$ = $CH_3$ (Schema 7)*

21.7 mg (25.7 μmol) Tubulysin A (**1**) wurden in 200 μl Methanol gelöst und bei Raumtemperatur in Abständen von 15 min dreimal mit etherischer Diazomethanlösung versetzt und über Nacht gerührt. Anschließend wurde der Reaktionsansatz zur Trockene gebracht. Die Reinigung erfolgte über PSC ($CH_2Cl_2$/MeOH 90/10) und lieferte 10.3 mg (46 %) 26 und 5.0 mg (23 %) Tubulysin-A-methylester **(20a).**

**DCI MS** (120 eV, $NH_3$): 872 $[M+H]^+$; **HRMS (DCI):** $C_{45}H_{69}N_5O_{10}S$: $[M+H]^+$ ber.: 872.4843 (gef.: 872.4818); **$^1$H-NMR** ($CD_3OD$, 400 MHz): δ = 2.6 (m, 1 H, Tut2-H), 2.86 (d, 2 H, Tut5-H), 7.17 (d, 2 H, Tut7-H), 6.85 (d, 2 H, Tut8-H), 1.19 (d, 3 H, Tut10-H), 3.65 (s, 3 H, Tut11-H), 3.78 (s, 3 H, Tut12-H)

## *Tubulysin-A-methylether (27a): R = i-$C_4H_9$, $R^1$ = $CH_3$ (Schema 7)*

1.6 mg (1.8 μmol) **26a** ($R^1$ = $CH_3$) wurden in 50 μl DMSO gelöst und mit 700 μl Phosphatpuffer (20 mM $KH_2PO_4$, pH = 7.3) versetzt. Der Reaktionsansatz wurde 5 min ins Ultraschallbad gestellt, anschließend wurden 72 μl Schweineleber-Esterase (Böhringer-Mannheim) zugegeben und 4 h bei 36°C gerührt. Zur Isolierung des Produktes wurde dreimal mit Ethylacetat extrahiert und die vereinigten organischen Phasen wurden zur Trockene gebracht. Eine Reinigung des Rohproduktes erfolgte über PSC ($CH_2Cl_2$/MeOH 90/10) und ergab 0.5 mg (32 %) **27a.**

**DCI MS** (120 eV, $NH_3$): 858 $[M+H]^+$; **HRMS (DCI):** $C_{44}H_{67}N_5O_{10}S$: $[M+H]^+$ ber.: 857.4687 (gef.: 858.4740); **$^1$H-NMR** ($CD_3OD$, 300 MHz): δ = 2.8 (m, 2 H, Tut5-H), 7.19 (d, 2 H, Tut7-H), 6.84 (d, 2 H, Tut8-H), 3.78 (s, 3 H, Tut11-H).

## *Iodierung von Tubulysin A (28a, 29a): R = i-$C_4H_9$, Hal = I (Schema 8)*

11.0 mg (13.1 μmol) Tubulysin A (**1**) wurden in 200 μl Methanol gelöst und mit 13.0 μl Iodmonochlorid-Lösung (13.1 μmol) versetzt. Der Reaktionsansatz wurde 15 Minuten bei Raumtemperatur gerührt und anschließend direkt über PSC ($CH_2Cl_2$/MeOH 90/10) gereinigt. Dabei wurden 3.1 mg (25 %) **28a** und 3.9 mg (27 %) **29a** erhalten.

**28a:**

**ESI MS** (1 eV): 970 $[M+H]^+$; **$^1$H-NMR** ($CD_3OD$, 300 MHz): δ = 2.58 (m, 1 H, Tut2-H), 1.71 (m, 1-H, Tut3a-H),

2.05 (m, 1-H, Tut3b-H), 4.28 (m, 1-H, Tut4-H), 2.82 (m, 2 H, Tut5-H), 7.10 (dd, 2 H, Tut7-H), 6.75 (d, 2 H, Tut8-H), 7.55 (d, 1 H, Tut12-H); **$^{13}$C-NMR** ($CD_3OD$, 75 MHz): δ = 181.2 (TutC1), 38.7 (TutC2), 39.5 (TutC3), 51.4 (TutC4), 40.6 (TutC5), 132.6 (TutC6), 131.5 (TutC7), 115.6 (TutC8), 156.5 (TutC9), 18.8 (TutC10), 84.4 (TutC11), 141.2 (TutC12).

**29a:**

**ESI MS** (1eV): 1096 $[M+H]^+$; **$^1$H-NMR** ($CD_3OD$, 600 MHz): δ = 2.58 (m, 1 H, Tut2-H), 1.73 (m, 1 H, Tut3a-H), 2.05 (m, 1 H, Tut3b-H), 4.25 (m, 1 H, Tut4-H), 2.75 (dd, 1 H, Tut5a-H), 2.86 (dd, 1 H, Tut5b-H), 7.61 (s, 2 H, Tut7, 12-H); **$^{13}$C-NMR** ($CD_3OD$, 75 MHz): δ = 181.1 (TutC1), 38.6 (TutC2), 39.6 (TutC3), 51.5 (TutC4), 40.1 (TutC5), 135.8 (TutC6), 141.5 (TutC7,12), 85.1 (TutC8,11), 155.2 (TutC9), 18.8 (TutC10).

## *Nitro-Tubulysin A (30a): R = i-$C_4H_9$ (Schema 8)*

Eine Lösung von 12.5 mg (14.8 μmol) Tubulysin A (1) in 400 μl Ethanol wurde mit 100 μl Eisessig und 20.5 mg (296.6 μmol) Natriumnitrit, gelöst in 100 μl Wasser, versetzt. Der Reaktionsansatz wurde zwei Tage bei Raumtemperatur gerührt und anschließend am Hochvakuum zur Trockene gebracht. Das Rohprodukt wurde mittels PSC ($CH_2Cl_2$/MeOH 90/10) gereinigt, wobei 9.8 mg (74 %) **30** erhalten wurden.

**IR** (KBr): $\tilde{\nu}$= 3411 $cm^{-1}$ (m), 2962 (m), 2932 (m), 2873 (w), 1741 (s), 1666 (vs), 1539 (s), 1492 (w), 1424 (w), 1370 (w), 1223 (s); **UV** (MeOH): $\lambda_{max}$ (lg ε) = 205 nm (4.56), 216 (sh, 4.42), 234 (sh, 4.19), 274 (3.77), 360 (3.43); **DCI MS** (120 eV, $NH_3$): 889 $[M+H]^+$; **$^1$H-NMR** ($CD_3OD$, 400 MHz): δ = 2.60 (m, 1 H, Tut2-H), 1.74 (m, 1 H, Tut3a-H), 2.09 (m, 1 H, Tut3b-H), 4.37 (ddd, 2 H, Tut4-H), 2.91 (dd, 1 H, Tut5a-H), 3.01 (dd, 1 H, Tut5b-H), 7.56 (dd, 1 H, Tut7-H), 7.07 (d, 1 H, Tut8-H), 1.27 (d, 3 H, Tut10-H), 7.97 (d, 1 H, Tut12-H); **$^{13}$C-NMR** ($CD_3OD$, 400 MHz): δ = 180.7 (TutC1), 38.5 (TutC2), 39.6 (TutC3), 51.0 (TutC4), 40.7 (TutC5), 132.0 (TutC6), 139.4 (TutC7), 120.8 (TutC8), 154.2 (TutC9), 18.8 (TutC10), 135.3 (TutC11), 126.5 (TutC12).

*Tubulysin-Derivate **31 und 32***

Ein Tubulysin-Derivat 31 kann man dadurch gewinnen, daß man das Nitro-Tubulysin A (30a) in Ethanol mit elementarem Wasserstoff mit einem Pd/C-Katalysator katalytisch reduziert.

Das gewonnen Tubulysin-Deriat 31 kann man mit Essigsäureanhydrid zu einem Tubulysin-Derivat 32 acylieren.

## *Tubulysin A-N-oxid (33a): R = i-$C_4H_9$ (Schema 9)*

9.9 mg (11.7 μmol) Tubulysin A (1) wurden in 200 μl Dichlormethan gelöst, mit 290 μl (11.7μmol) *m*-CPBA-Lösung (10 mg/ml Dichlormethan) versetzt und 30 Minuten bei Raumtemperatur gerührt. Nachdem der Reaktionsansatz reduziert wurde, erfolgte direkt die Reinigung mittels PSC ($CH_2Cl_2$/Methanol 85/15), wobei 5.2 mg (52 %) **33a** erhalten wurden.

**ESI MS** (1 eV): 860 $[M+H]^+$.

*Tubulysin-Derivat **34***

Ein Tubulysin-Derivat 34 kann man dadurch gewinnen, daß man Tubulysin A-N-oxid (33a) bei etwa 75 ˚C mit Essigsüreanhydrid behandelt.

Abkürzungen

| Abkürzung | Name |
|---|---|
| $C_5Cl_5NF$-Triflat | *N*-Fluorpentachlorpyridinium-Triflat |
| $CH_3CN$ | Acetonitril |
| DAST | Diethylaminoschwefeltrifluorid |

(fortgesetzt)

| Abkürzung | Name |
|---|---|
| DMAP | Dimethylaminopyridin |
| EDC | *N*-Ethyl-*N'*-(3-dimethylaminopropyl)-carbodiimid |
| IC1 | Iodmonochlorid |
| *m*-CPBA | meta-Chlorperbenzoesäure |
| $Me_3SiCl$ | Trimethylchlorsilan |
| $NaCNBH_3$ | Natriumcyanoborhydrid |
| NBS | *N*-Bromsuccinimid |
| NMO | *N*-Methyl-morpholin-*N*-oxid |
| *p*-$CH_3$-$C_6H_4SO_2OH$ | *para*-Toluolsulfonsäure |
| TFA | Trifluoressigsäure |
| THF | Tetrahydrofuran |
| TPAP | *tetra*-Propylanmnoniumperruthenat |

1. Verbindung der folgenden allgemeinen Formel I (Tubulysin):

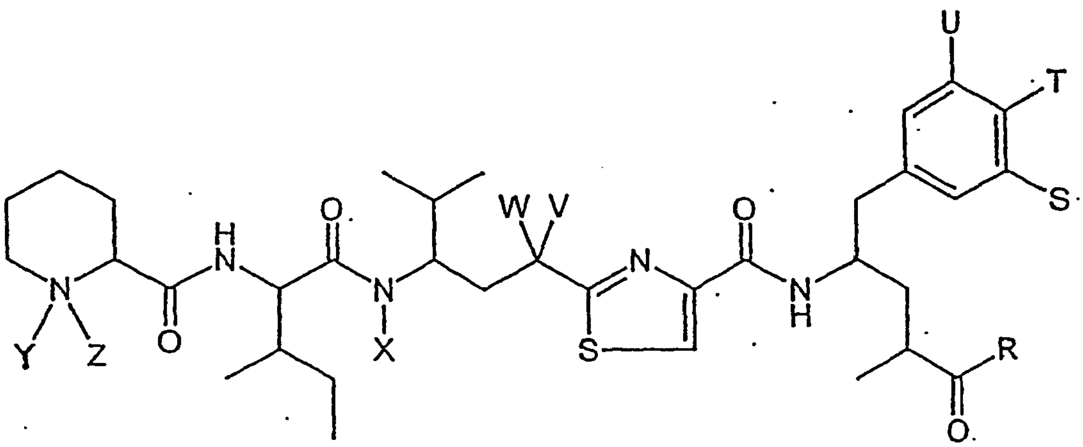

mit den folgenden Bedeutungen für R, $R^1$, $R^2$, $R^3$, $R^4$, $R^5$, $R^6$, $R^7$, $R^8$, $R^9$, $R^{10}$, $R^{11}$, S, T, U, V, W, X, Y und Z:
R = H, Alkyl, Aryl, $OR^1$, $NR^1R^2$ oder

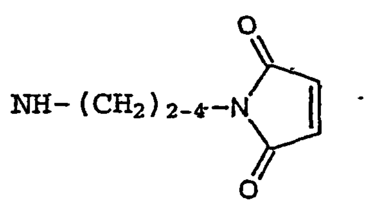

$R^1$ = H, Alkyl oder Aryl
$R^2$ = H, Alkyl oder Aryl
S = H, Hal, $NO_2$ oder $NHR^3$
U = H, Hal, $NO_2$ oder $NHR^3$
$R^3$ = H, HCO oder Alkyl-CO
T = H oder $OR^4$
$R^4$ = H, Alkyl, Aryl, $COR^5$, P(O) $(OR^6)_2$ oder $SO_3R^6$
$R^5$ = Alkyl, Alkenyl, Aryl oder Heteroaryl
$R^6$ = H, Alkyl oder Metallion

V = H, $OR^7$, Hal oder (mit W = O)
$R^7$ = H, Alkyl oder $COR^8$
$R^8$ = Alkyl, Alkenyl oder Aryl
W = H oder Alkyl oder (mit V) O
X = H, Alkyl, Alkenyl oder $CH_2OR^9$
$R^9$ = H, Alkyl, Alkenyl, Aryl oder $COR^{10}$
$R^{10}$ = Alkyl, Alkenyl, Aryl oder Heteroaryl
Y = (für Z = $CH_3$ oder $COR^{11}$) freies Elektronenpaar oder (für Z = $CH_3$) O
$R^{11}$ = Alkyl, $CF_3$ oder Aryl und/oder
Z = (für Y = O oder freies Elektronenpaar) $CH_3$ oder (für Y = freies Elektronenpaar) $COR^{11}$.

2. Verbindung nach Ausführungsform 1 mit
R, $R^1$, $R^4$, $R^5$, $R^8$, $R^9$, $R^{10}$ und/oder $R^{11}$ = unsubstituiertes oder substituiertes Phenyl, insbesondere $C_{1-4}$-Alkyl-substituiertes Phenyl
$R^5$ = $C_{1-4}$-Alkyl, $C_{2-6}$-Alkenyl oder Pyridyl
$R^5$ und/oder X = $C_{2-4}$-Alkenyl
$R^6$ = Alkalimetall-Ion, insbesondere Na-Ion, oder Erdalkalimetall-Ion
$R^8$ und/oder $R^9$ = $C_{2-4}$-Alkenyl und/oder
$R^{10}$ = $C_{2-6}$-Alkenyl, insbesondere $C_{2-4}$-Alkenyl, oder Pyridyl.

3. (Schema 1) Verfahren zur Herstellung einer Verbindung der allgemeinen Formel I gemäß Ausführungsform 1 (Typ 7) mit R = $OR^1$, R1 = H, S = U = H, T = H oder OH, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = H, Y = freies Elekronenpaar und Z = $CH_3$, bei dem man eine Verbindung der folgenden allgemeinen Formel II (Typ 1, 2, 3, 4, 5 oder 6):

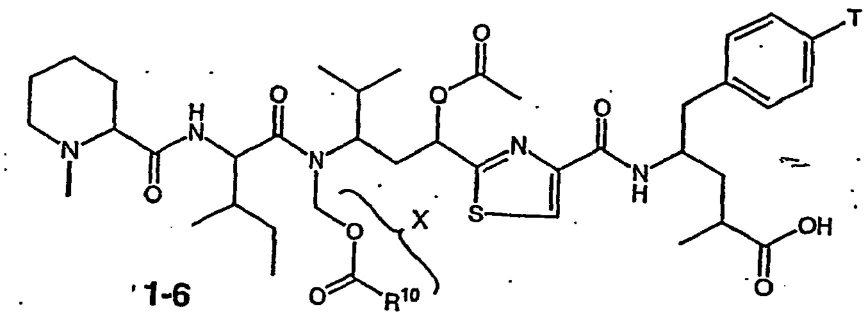

mit X = $CH_2O_R{}^9$, $R^9$ = $COR^{10}$, $R^{10}$ = Alkyl, insbesondere $C_{1-6}$-Alkyl, und im übrigen den vorstehend angegebenen Bedeutungen einer Esterspaltung in saurem Medium unterwirft und die Verbindung der allgemeinen Formel I gemäß Ausführungsform 1 mit den angegebenen Bedeutungen gewinnt.

4. Verfahren nach Ausführungsform 3, bei dem man die Esterspaltung in einem organischen Lösungsmittel, insbesondere Dioxan, in Gegenwart einer Säure, insbesondere Chlorwassertoff, und/oder bei erhöhter Temperatur durchführt.

5. (Schema 1) Verfahren zur Herstellung einer Verbindung der allgemeinen Formel I gemäß Ausführungsform 1 (Typ 8) mit R = $OR^1$, $R^1$ = H, S = U = H, T = H oder OH, V = $OR^7$, $R^7$ = $COR^8$, $R^8$ = Alkyl, vorzugsweise $C_{1-4}$-Alkyl, insbesondere Methyl, W = H, X = H, Y = freies Eletronenpaar und Z = $CH_3$, bei dem man eine Verbindung der allgemeinen Formel II gemäß Ausführungsform 3 (Typ 1, 2, 3, 4, 5 oder 6) mit X = $CH_2OR^9$, $R^9$ = $COR^{10}$, $R^{10}$ = Alkyl, vorzugsweise $C_{1-6}$-Alkyl, und im übrigen den vorstehend angegebenen Bedeutungen einer Acetal-Spaltung unterwirft und die Verbindung der allgemeinen Formel I gemäß Ausführungsform 1 mit den angegebenen Bedeutungen gewinnt.

6. Verfahren nach Ausführungsform 5, bei dem man die Acetal-Spaltung in saurem Milieu, insbesondere in Gegenwart von Salzsäure, und/oder bei erhöhter Temperatur durchführt.

7. (Schema 1) Verfahren zur Herstellung einer Verbindung der allgemeinen Formel I gemäß Ausführungsform 1 (Typ 9) mit R = $OR^1$, $R^1$ = H, S = U = H, T = H oder OH, V = $OR^7$, $R^7$ = H, W = H, X = $CH_2OR^9$, $R^9$ = $COR^{10}$, $R^{10}$ = Alkyl, insbesondere $C_{1-6}$-Alkyl, Y = freies Elektronenpaar und Z = $CH_3$, bei dem man eine Verbindung der allgemeinen Formel II gemäß Ausführungsform 3 (Typ 1, 2, 3, 4, 5 oder 6) mit V = $OR^7$, $R^7$ = $COR^8$, $R^8$ = Alkyl, vorzugsweise $C_{1-4}$-Alkyl, insbesondere Methyl, und im übrigen den vorstehend angegebenen Bedeutungen einer Esterspaltung in schwach alkalischem Medium unterwirft und die Verbindung der allgemeinen Formel I gemäß Ausführungsform 1 it den angegebenen Bedeutungen gewinnt.

8. Verfahren nach 7, bei dem man die Esterspaltung in einem organischen Medium, insbesondere einem hydrophilen organischen Lösungsmittel, vorzugsweise einem Alkohol, insbesondere Methanol, in Gegenwart einer schwachen Base durchführt, insbesondere $NH_3$.

9. (Schema 1) Verfahren zur Herstellung einer Verbindung der allgemeinen Formel I gemäß Ausführungsform 1 (Typ 10) mit R = $OR^1$, $R^1$ = H, S = U = H, T = H oder OH, V = $OR^7$, $R^7$ = H, W = H, X = H, Y = freies Elektronenpaar und Z = $CH_3$, bei dem man eine Verbindung der allgemeinen Formel I gemäß Ausführungsform 3 (Typ 1, 2, 3, 4, 5 oder 6) mit V = $OR^7$, $R^7$ = $COR^8$, $R^8$ = Alkyl, vorzugsweise $C_{1-4}$-Alkyl, insbesondere Methyl, X = $CH_2OR^9$, $R^9$ = $COR^{10}$, $R^{10}$ = Alkyl, insbesondere $C_{1-6}$-Alkyl, und im übrigen den vorstehend angegebenen Bedeutungen einer doppelten Esterspaltung in stark alkalischem Medium unterwirft und die Verbindung der allgemeinen Formel I gemäß Ausführungsform 1 mit den angegebenen Bedeutungen gewinnt.

10. Verfahren nach Ausführungsform 9, bei dem man die doppelte Esterspaltung in einem organischen Medium, insbesondere in einem hydrophilen organischen Lösungsmittel, vorzugsweise Alkohol, insbesondere Methanol, in Gegenwart einer starken Base durchführt, insbesondere eines Alkalimetallhydroxids, vorzugsweise von Natriumhydroxid.

11. (Schema 1) Verfahren zur Herstellung einer Verbindung der folgenden allgemeinen Formel III (Typ 11):

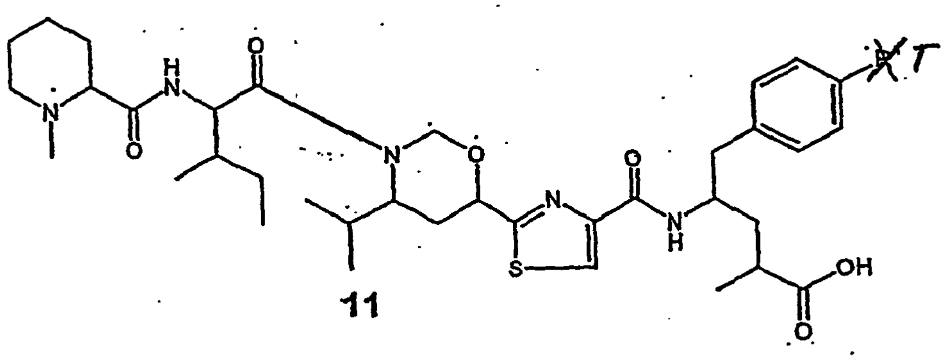

11

mit R = $OR^1$, $R^1$ = H, S = U = H, T = H oder $OR^4$, $R^4$ = H, V mit X = $CH_2O$-Brücke, W = H, Y = freies Elektronenpaar und Z = $CH_3$ in der allgemeinen Formel I gemäß Ausführungsform 1, bei dem man eine Verbindung der allgemeinen Formel II gemäß Ausführungsform 3 (Typ 1, 2, 3, 4, 5 oder 6) mit X = $CH_2OR^9$, $R^9$ = $COR^{10}$, $R^{10}$ = Alkyl, insbesondere $C_{1-6}$-Alkyl, V = $OR^7$, $R^7$ = $COR^8$, $R^8$ = Alkyl, vorzugsweise $C_{1-4}$-Alkyl, insbesondere Methyl, und im übrigen den vorstehend angegebenen Bedeutungen einer Ringbildung unter doppelter Esterspaltung in saurem Medium unterwirft und die Verbindung der vorstehenden allgemeinen Formel mit den angegebenen Bedeutungen gewinnt.

12. Verfahren nach Ausführungsform 11, bei dem man die Ringbildung in wässerigem Medium, in Gegenwart einer anorganischen Säure, vorzugsweise Salzsäure, und unter Erhitzen durchführt.

13. (Schema 2) Verfahren zur Herstellung einer Verbindung der allgemeinen Formel I gemäß Ausführungsform 1 (Typ 12) mit R = $OR^1$, $R^1$ = H, S = U = H, T = H oder $OR^4$, $R^4$ = $COR^5$, $R^5$ = Alkyl, insbesondere $C_{1-6}$-Alkyl, Alkenyl, insbesondere $C_{2-6}$-Alkenyl, Aryl oder Heteroaryl, V = $OR^7$, $R^7$ = $COR^8$, $A^8$ = Alkyl, vorzugsweise $C_{1-4}$-Alkyl, insbesondere Methyl, W = H, X = $CH_2OR^9$, $R^9$ = $COR^{10}$, $R^{10}$ = $R^5$, Y = freies Elektronenpaar und Z = $CH_3$; bei dem man eine Verbindung der folgenden allgemeinen Formel IV (Typ 7):

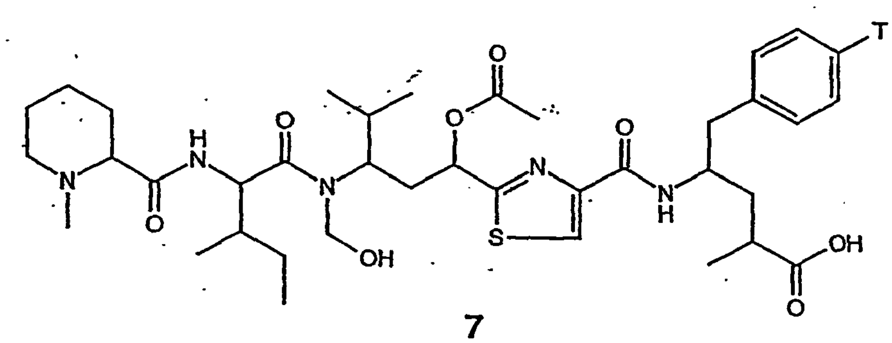

7

mit X = $CH_2OR^9$, $R^9$ = H und im übrigen den vorstehend angegebenen Bedeutungen einer Acylierung unterwirft und eine Verbindung der allgemeinen Formel I gemäß Ausführungsform 1 mit den angegebenen Bedeutungen gewinnt.

14. Verfahren nach Ausführungsform 13, bei dem man mit einem Acylhalogenid, insbesondere Acylchlorid, und/oder in Gegenwart einer.schwachen Base acyliert, insbesondere einer schwachen organischen Base, vorzugsweise eines tertiären Amins, insbesondere Triethylamin.

15. (Schema 2) Verfahren zur Herstellung einer Verbindung der allgemeinen Formel I gemäß Ausführungsform 1 (Typ 13) mit R = $OR^1$, $R^1$ = H, S = U = H, T = H oder $OR^4$, $R^4$ = H, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl, insbesondere $C_{1-6}$-Alkyl, Alkenyl, insbesondere $C_{2-6}$-Alkenyl, Aryl oder Heteroaryl, Y = freies Elektronenpaar und Z = $CH_3$, bei dem man bei einem Produkt des Verfahrens gemäß <-> 13 mit T = $OR^4$, $R^4$ = $COR^5$ und $R^5$ = Alkyl, insbesondere $C_{1-6}$-Alkyl, Alkenyl, insbesondere $C_{2-6}$-Alkenyl, Aryl oder Heteroaryl und im übrigen den vorstehend angegebenen Bedeutungen in alkalischem Medium verseift und eine Verbindung der allgemeinen Formel I gemäß Ausführungsform 1 mit den angegebenen Bedeutungen gewinnt.

16. Verfahren nach Ausführungsform 15, bei dem man mit Ammoniak verseift.

17. (Schema 3) Verfahren zur Herstellung einer Verbindung der allgemeinen Formel I gemäß Ausführungsform 1 (Typ 14) mit R = $OR^1$, $R^1$ = H, S = U = H, T = H oder OH, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = Alkyl, insbesondere $C_{1-4}$-Alkyl, Alkenyl oder Aryl, Y = freies Elektronenpaar und Z = $CH_3$, bei dem man eine Ausgangsverbindung des Verfahrens gemäß Ausführungsform 3 (Typ 1, 2, 3, 4, 5 oder 6) einer Esterspaltung unterwirft und alkyliert und eine Verbindung der allgemeinen Formel I gemäß Ausführungsform 1 mit den angegebenen Bedeutungen gewinnt.

18. Verfahren nach Ausführungsform 7, bei dem man mit einem Alkylierungsmittel der Formel $R^9OH$ mit $R^9$ = Alkyl, insbesondere $C_{1-4}$-Alkyl, Alkenyl oder Aryl umsetzt.

19. Verfahren nach 17 oder 18, bei dem man in Gegenwart von p-$CH_3$-$C_6H_4SO_2OH$ in Tetrahydrofuran (THF) bei erhöhter Temperatur umsetzt.

20. (Schema 4) Verfahren zur Herstellung einer Verbindung der allgemeinen Formel I gemäß Ausführungsform 1 (Typ 15) mit R = $OR^1$, $R^1$ = H, S = U = H, T = H oder $OR^4$, $R^4$ = H, V = $OR^7$, $R^7$ = H oder $COR^8$, $R^8$ = Alkyl, vorzugsweise $C_{1-4}$-Alkyl, insbesondere Methyl, W = H, X = $CH_3$, Y = freies Elektronenpaar und Z = $CH_3$, bei dem man ein Produkt des Verfahrens gemäß Ausführungsform 3 (Typ 7) mit X = $CH_2OR^9$, $R^9$ = H und im übrigen den vorstehend angegebenen Bedeutungen einer Reduktion unterwirft und die Verbindung der allgemeinen Formel I gemäß Ausführungsform 1 mit den angegebenen Bedeutungen gewinnt.

21. Verfahren nach Ausführungsform 20, bei dem man die Reduktion mit $NaCNBH_3$ und Trifluoressigsäure in Methanol (MeOH) durchführt.

22. (Schema 4) Verfahren zur Herstellung einer Verbindung der allgemeinen Formel I gemäß Ausführungsform 1 (Typ 15) mit R = $OR^1$, $R^1$ = H, S = U = H, T = H oder $OR^4$, $R^4$ = H, V = $OR^7$, $R^7$ = H oder $COR^8$, $R^8$ = Alkyl,

insbesondere $C_{1-4}$-Alkyl, insbesondere Methyl, W = H, X = $CH_3$, Y = freies Elektronenpaar und Z = $CH_3$, bei dem man eine Verbindung der allgemeinen Formel gemäß Ausführungsform 11 (Typ 11) einer Ringöffnung unter Reduktion bzw. Reduktion unter Ringöffnung unterwirft und eine Verbindung der allgemeinen Formel I gemäß Ausführungsform 1 mit den angegebenen Bedeutungen gewinnt.

23. Verfahren nach Ausführungsform 20, bei dem man in Gegenwart von $NaCNBH_3$ in Acetonitril und, $Me_3SiCl$ und ($CH_3CN$) umsetzt.

24. (Schema 5) Verfahren zur Herstellung einer Verbindung der allgemeinen Formel I gemäß Ausführungsform 1 (Typ 16) mit R = $OR^1$, $R^1$ = H, S = U = H, T = H oder OH, V = $OR^7$, $R^7$ = $COR^8$, $R^8$ = Alkyl, insbesondere $C_{1-4}$-Alkyl, Alkenyl oder Aryl, W = H, X = $CH_2OR^9$, $R^9$ = $COR^{10}$, $R^{10}$ = Alkyl, insbesondere $C_{1-6}$-Alkyl, oder Alkenyl, Y = freies Elektronenpaar und Z = $CH_3$, bei dem man ein Produkt eines Verfahrens gemäß Ausführungsform 7 (Typ 9) mit V = $OR^7$ und $R^7$ = H und im übrigen den vorstehend angegebenen Bedeutungen einer Acylierung unterwirft und die Verbindung der allgemeinen Formel I gemäß Ausführungsform 1 mit den angegebenen Bedeutungen gewinnt.

25. Verfahren nach Ausführungsform 24, bei dem man die Acylierung mit einem Acylhalogenid der Formel $R^8COCl$ mit $R^8$ = Alkyl, insbesondere $C_{1-4}$-Alkyl, Alkenyl oder Aryl, insbesondere Acylchlorid, und/oder in Gegenwart einer Base durchführt, insbesondere einer organischen Base, vorzugsweise eines Trialkylamins, insbesondere Triethylamin.

26. (Schema 5) Verfahren zur Herstellung einer Verbindung der allgemeinen Formel I gemäß. Ausführungsform 1 (Typ 17) mit R = $OR^1$, $R^1$ = H, S = U = H, T = H oder $OR^4$, $R^4$ = H, V = H oder F, W = H, X = $CH_2OR^9$, $R^9$ = $COR^{10}$, $R^{10}$ = Alkyl, insbesondere $C_{1-6}$-Alkyl, oder Alkenyl, Y = freies Elektronenpaar und Z = $CH_3$, bei dem man ein Produkt eines Verfahrens gemäß Ausführungsform 7 (Typ 9) mit V = $OR^7$ und $R^7$ = H und im übrigen den vorstehend angegebenen Bedeutungen einer katalytischen Hydrierung oder einer Fluorierung unterwirft und die Verbindung der allgemeinen Formel I gemäß Ausführungsform 1 mit und die Verbindung der allgemeinen mit den angegebenen Bedeutungen gewinnt.

27. Verfahren nach Ausführungsform 26, bei dem man für V = H die Hydrierung mit Palladium/Kohlenstoff in Gegenwart von Essigsäure und für V = F die Fluorierung mit DAST in Tetrahydrofuran durchführt.

28. (Schema 5) Verfahren zur Herstellung einer Verbindung der allgemeinen Formel I gemäß Ausführungsform 1 (Typ 18) mit R = $OR^1$, $R^1$ = H, S = U = H, T = H oder $OR^4$, $R^4$ = H, V mit W = O, X = $CH_2OR^9$, $R^9$ = $COR^{10}$, $R^{10}$ = Alkyl, insbesondere $C_{1-6}$-Alkyl , oder Alkenyl, Y = freies Elektronenpaar und Z = $CH_3$, bei dem man ein Produkt eines Verfahrens gemäß Ausführungsform 7 (Typ 9) mit V = $OR^7$ und $R^7$ = H und im übrigen den vorstehend angegebenen Bedeutungen einer Oxidation unter Bildung eines Ketons unterwirft und eine Verbindung der allgemeinen Formel I gemäß Ausführungsform 1 mit den angegebenen Bedeutungen gewinnt.

29. Verfahren nach Ausführungsform 28, bei dem man die Oxidation in Gegenwart von TPAP und in Dichlormethan NMO durchführt.

30. (Schema 5) Verfahren zur Herstellung einer Verbindung der allgemeinen Formel I gemäß Ausführungsform 1 (Typ 19) mit R = $OR^1$, $R^1$ = H, S = U = H, T = H oder OH, V $OR^7$, $R^7$ = H, W = Alkyl, insbesondere $C_{1-4}$-Alkyl, X = $CH_2OR^9$, $R^9$ = $COR^{10}$, $R^{10}$ = Alkyl, insbesondere $C_{1-6}$-Alkyl, oder Alkenyl, Y = freies Elektronenpaar und Z = $CH_3$, bei dem man ein Produkt eines Verfahrens gemäß Ausführungsform 28 oder 29 (Typ 18) mit einer Grignard-Verbindung zur Verbindung der allgemeinen Formel I gemäß Ausführungsform 1 mit den angegebenen Bedeutungen umsetzt.

31. Verfahren nach Ausführungsform 30, bei dem man die Umsetzung mit einer magnesiumorganischen Verbindung der Formel WMgHal mit W = Alkyl und insbesondere $C_{1-4}$-Alkyl durchführt.

32. (Schema 5) Verfahren zur Herstellung einer Verbindung der allgemeinen Formel I gemäß Ausführungsform 1 (Typ 19) mit R = $OR^1$, $R^1$ = H, S = U = H, T = H oder OH, V = $OR^7$, $R^7$ = H, W = Alkyl und insbesondere $C_{1-4}$-Alkyl, X = $CH_2OR^9$, $R^9$ = $COR^{10}$, $R^{10}$ = Alkyl, insbesondere $C_{1-6}$-Alkyl, oder Alkenyl, Y = freies Elektronenpaar und Z = $CH_3$, bei dem man

(i) in einer ersten Stufe ein Verfahren gemäß Ausführungsform 28 oder 29 durchführt und danach
(ii) in einer zweiten Stufe ein Verfahren gemäß Ausführungsform 30 oder 31 durchführt und eine Verbindung

der allgemeinen Formel I gemäß Ausführungsform 1 mit den angegebenen Bedeutungen gewinnt.

33. (Schema 6) Verfahren zur Herstellung einer Verbindung der allgemeinen Formel I gemäß Ausführungsform 1 (Typ 20) mit R = $OR^1$, $R^1$ = Alkyl, insbesondere $C_{1-4}$-Alkyl, oder Alkenyl, S = U = H, T = H oder $OR^4$, $R^4$ = H, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl, insbesondere $C_{1-6}$-Alkyl, Alkenyl, insbesondere $C_{2-6}$-Alkenyl, Aryl oder Heteroaryl, Y = freies Elektronenpaar und Z = $CH_3$, bei dem man eine Ausgangsverbindung eines Verfahrens gemäß Ausführungsform 3 (Typ 1, 2, 3, 4, 5 oder 6) oder ein Produkt eines Verfahrens gemäß Ausführungsform 15 (Typ 13) einer Alkylierung oder Alkenylierung unterwirft und eine Verbindung der allgemeinen Formel I gemäß Ausführungsform 1 mit den angegebenen Bedeutungen gewinnt.

34. Verfahren nach Ausführungsform 33, bei dem man die Alkylierung oder Alkenylierung in Gegenwart von EDC, $R^1OH$ mit $R^1$ = Alkyl, insbesondere $C_{1-4}$-Alkyl, oder Alkenyl und DMAP in Methylenchlorid durchführt.

35. (Schema 6) Verfahren zur Herstellung einer Verbindung der allgemeinen Formel I gemäß Ausführungsform 1 (Typ 21) mit R = $NHR^1$, $NH-NR^1R^2$, $NHOR^1$ oder $NH(CH_2)_{2-4}NR^1R^2$, $R^1$ und $R^2$ unabhängig voneinander = H, Alkyl, insbesondere $C_{1-6}$-Alkyl, oder Aryl, S = U = H, T = H oder $OR^4$, $R^4$ = H, V = $OR^7$, $R^7$ = $COR^8$, $R^8$ = Alkyl, vorzugsweise $C_{1-4}$-Alkyl, insbesondere Methyl, W = H, X = $CH_2OR_9$, $R^9$ = $COR^{10}$, $R^{10}$ = Alkyl, insbesondere $C_{1-6}$-Alkyl, Alkenyl, insbesondere $C_{2-6}$-Alkenyl, Aryl oder Heteroaryl, Y = freies Elektronenpaar und Z = $CH_3$, bei dem man eine Ausgangsverbindung eines Verfahrens gemäß Ausführungsform 3 (Typ 1, 2, 3, 4, 5 oder 6) oder ein Produkt eines Verfahrens gemäß Ausführungsform 15 (Typ 13) mit einer Verbindung der Formel RH einer Aminierung unterwirft, wobei R die angegebenen Bedeutungen besitzt, und eine Verbindung der allgemeinen Formel I gemäß Ausführungsform 1 mit den angegebenen Bedeutungen gewinnt.

36. Verfahren nach Ausführungsform 35, bei dem man die Umsetzung

(i) in Gegenwart von EDC in Methylenchlorid oder
(ii) in Gegenwart von i-Butylchlorformiat und Triethylamin in THF durchführt.

37. (Schema 6) Verfahren zur Herstellung einer Verbindung der allgemeinen Formel I gemäß Ausführungsform 1 (Typ 22) mit R = Alkyl, insbesondere $C_{1-4}$-Alkyl, oder Alkenyl, S = U = H, T = H oder $OR^4$, $R^4$ = H, V = $OR^7$, $R^7$ = $COR^8$, $R^8$ = Alkyl, vorzugsweise $C_{1-4}$-Alkyl; insbesondere Methyl, W = H, X = $CH_2OR^9$, $R^9$ = $COR^{10}$, $R^{10}$ = Alkyl, insbesondere $C_{1-6}$-Alkyl, Alkenyl, insbesondere $C_{2-6}$-Alkenyl, Aryl oder Heteroaryl, Y = freies Elektronenpaar und Z = $CH_3$, bei dem man eine Ausgangsverbindung eines Verfahrens gemäß Ausführungsform 3 (Typ 1, 2, 3, 4, 5 oder 6) oder ein Produkt eines Verfahrens gemäß Ausführungsform 15 (Typ 13) mit einer lithiumorganischen Verbindung der Formel RLi mit der angegebenen Bedeutung für R zu der Verbindung der allgemeinen Formel I gemäß Ausführungsform 1 mit den angegebenen Bedeutungen umsetzt.

38. (Schema 6) Verfahren zur Herstellung einer Verbindung der allgemeinen Formel I gemäß Ausführungsform 1 (Typ 23) mit R = Aminorest von 1-(2-Amino-$C_{2-4}$-alkyl)-pyrrol-2,5-dion, S = U = H, T = H oder $OR^4$, $R^4$ = H, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ =$COR^{10}$, $R^{10}$ = Alkyl, insbesondere $C_{1-6}$-Alkyl, Alkenyl, insbesondere $C_{2-6}$-Alkenyl, Aryl oder Heteroaryl, Y = freies Elektronenpaar und Z = CH3, bei dem man eine Ausgangsverbindung eines Verfahrens gemäß Ausführungsform 3 (Typ 1, 2, 3, 4, 5 oder 6) oder ein Produkt eines Verfahrens gemäß Ausführungsform 15 (Typ 13) einer Aminierung mit 1-(2-Amino-$C_{2-4}$-alkyl)-pyrrol-2,5-dion unterwirft und die Verbindung der allgemeinen Formel I gemäß Ausführungsform 1 mit den angegebenen Bedeutungen gewinnt.

39. Verfahren nach Ausführungsform 38, bei dem man die Aminierung in Gegenwart von EDC in Methylenchlorid durchführt.

40. (Schema 7) Verfahren zur Herstellung einer Verbindung der allgemeinen Formel I gemäß Ausführungsform 1 (Typ 24) mit R = $OR^1$, $R^1$ = H, S = U = H, T = $OR^4$, $R^4$ = $P(O)(OR^6)_2$ mit $R^6$ = H oder Alkyl, insbesondere $C_{1-4}$-Alkyl, oder $R^4$ = $SO_3R^6$ mit $R^6$ = H, V = $OR^7$, $R^7$= $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl, insbesondere $C_{1-6}$-Alkyl, Alkenyl, insbesondere $C_{2-6}$-Alkenyl, Aryl oder Heteroaryl, Y = freies Elektronenpaar und Z = $CH_3$, bei dem man

(i) eine Ausgangsverbindung (Typ 1, 2 oder 3) gemäß Ausführungsform 3 oder
(ii) ein Produkt eines Verfahrens gemäß Ausführungsform 15 (Typ 13) mit

(a) einer Verbindung der Formel $P(O)(OR^6)_2OH$ mit $R^6$ = H oder Alkyl, insbesondere $C_{1-4}$-Alkyl, oder
(b) $SO_3$
umsetzt und die Verbindung der allgemeinen Formel I gemäß Ausführungsform 1 mit den angegebenen Bedeutungen gewinnt.

41. Verfahren nach Ausführungsform 40, bei dem man die Variante (a) in Gegenwart von $I_2$ und Pyridin in Methylenchlorid durchführt.

42. Verfahren nach Ausführungsform 40, bei dem man die Variante (b) mit Pyridin-$SO_3$ durchführt.

43. (Schema 7) Verfahren zur Herstellung einer Verbindung der allgemeinen Formel I gemäß Ausführungsform 1 (Typ 25) mit R = $OR^1$, $R^1$ = H, S = U = H, T = $OR^4$, $R^4$ = $COR^5$, $R^5$ = Alkyl, insbesondere $C_{1-4}$-Alkyl, Alkenyl oder N $(R^{12})_2$ , $R^{12}$ = Alkyl, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl, insbesondere $C_{1-6}$-Alkyl, Alkenyl, insbesondere $C_{2-6}$-Alkenyl, Aryl oder Heteroaryl, bei dem man

(i) eine Ausgangsverbindung (Typ 1, 2 oder 3) gemäß Ausführungsform 3 oder
(ii) ein Produkt eines Verfahrens gemäß Ausführungsform 15 (Typ 13) einer Acylierung unterwirft und die Verbindung der allgemeinen Formel I gemäß Ausführungsform 1 mit den angegebenen Bedeutungen gewinnt.

44. Verfahren nach Ausführungsform 43, bei dem man die Acylierung mit einem Acylhalogenid der Formel $R^5COCl$ mit $R^5$ = Alkyl, insbesondere $C_{1-4}$-Alkyl , Alkenyl oder $N(R^{12})_2$ und $R^{12}$ = Alkyl, insbesondere mit einem Acylchlorid, in Gegenwart einer organischen Base, insbesondere eines Trialkylamins, vorzugsweise Triethylamin, in einem organischen Lösungsmittel durchführt, insbesondere THF.

45. (Schema 7) Verfahren zur Herstellung einer Verbindung der allgemeinen Formel I gemäß Ausführungsform 1 (Typ 26) mit R = $OR^1$, $R^1$ = Alkyl, insbesondere $C_{1-4}$-Alkyl, oder Alkenyl, S = U = H, T = $OR^4$ , $R^4$ = Alkyl, insbesondere $C_{1-4}$-Alkyl, oder Alkenyl, V = $OR^7$, $R^7$= $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl, insbesondere $C_{1-6}$-Alkyl, Alkenyl, insbesondere $C_{2-6}$-Alkenyl, Aryl oder Heteroaryl, Y = freies Elektronenpaar und Z = $CH_3$, bei dem man

(i) eine Ausgangsverbindung (Typ 1, 2 oder 3) gemäß Ausführungsform 3 oder
(ii) ein Produkt eines Verfahrens gemäß Ausführungsform 15 (Typ 13) einer Alkylierung unterwirft und die Verbindung der allgemeinen Formel I gemäß Ausführungsform 1 mit den angegebenen Bedeutungen gewinnt.

46. Verfahren nach Ausführungsform 45, bei dem man mit einem Alkyliodid der Formel $R^4I$ mit $R^4$ = Alkyl, insbesondere $C_{1-4}$-Alkyl, oder Alkenyl in Gegenwart einer schwachen Base, insbesondere $Ag_2O$, in einem organischen Lösungsmittel alkyliert, insbesondere Methylenchlorid.

47. Verfahren nach Ausführungsform 45, bei dem man mit Diazomethan in einem organischen Lösungsmittel methyliert, insbesondere Methanol.

48. (Schema 7) Verfahren zur Herstellung einer Verbindung der allgemeinen Formel I gemäß Ausführungsform 1 (Typ 27) mit R = $OR^1$, $R^1$ = H, S = U = H, T = $OR^4$, $R^4$ = Alkyl, insbesondere $C_{1-4}$-Alkyl, oder Alkenyl, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl, insbesondere $C_{1-6}$-Alkyl, Alkenyl, insbesondere $C_{2-6}$-Alkenyl, Aryl oder Heteroaryl, Y = freies Elektronenpaar und Z = $CH_3$, bei dem man ein Produkt des Verfahrens gemäß Ausführungsform 45, 46 oder 47 (Typ 26) enzymatisch einer partiellen Dealkylierung oder Dealkenylierung unterwirft und die Verbindung der allgemeinen Formel I gemäß Ausführungsform 1 mit den angegebenen Bedeutungen gewinnt.

49. Verfahren nach Ausführungsform 48, bei dem man als Enzym eine Esterase verwendet, insbesondere Schweineleber-Esterase.

50. (Schema 7) Verfahren zur Herstellung einer Verbindung der allgemeinen Formel I gemäß Ausführungsform 1 (Typ 27) R = $OR^1$, $R^1$ = H, S = U = H, T = $OR^4$, $R^4$ = Alkyl, insbesondere $C_{1-4}$-Alkyl, oder Alkenyl, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl, insbesondere $C_{1-6}$-Alkyl, Alkenyl, insbesondere $C_{2-6}$-Alkenyl, Aryl oder Heteroaryl, bei dem man

(a) in einer ersten Stufe

(i) eine Ausgangsverbindung (Typ 1, 2 oder 3) gemäß Ausführungsform 3 oder
(ii) ein Produkt eines Verfahrens gemäß Ausführungsform 15 (Typ 13) einem Verfahren gemäß Ausführungsform 45, 46 oder 47 unterwirft und

(b) in einer zweiten Stufe ein Verfahren gemäß Ausführungsform 48 oder 49 durchführt und die Verbindung der allgemeinen Formel I gemäß Ausführungsform 1 mit den angegebenen Bedeutungen gewinnt.

51. (Schema 8) Verfahren zur Herstellung einer Verbindung der allgemeinen Formel I gemäß Ausführungsform 1 (Typ 28 und ggf. 29) mit R = $OR^1$, $R^1$ = H, S = H oder Hal, T = $OR^4$, $R^4$ = H, U = Hal, V = $OR^7$, $R^7$ = $COR^8$, $R^8$ = Alkyl, vorzugsweise $C_{1-4}$-Alkyl, insbesondere Methyl, W = H, X = $CH_2OR^9$ , $R^9$ = $COR^{10}$, $R^{10}$ = Alkyl, insbesondere $C_{1-6}$-Alkyl, Alkenyl, insbesondere $C_{2-6}$-Alkenyl, Aryl oder Heteroaryl, bei dem man

(i) eine Ausgangsverbindung (Typ 1, 2, 3, 4, 5 oder 6) gemäß Ausführungsform 3 oder
(ii) ein Produkt eines Verfahrens gemäß Ausführungsform 15 (Typ 13) einer Halogenierung oder Dihalogenierung in ortho-Stellung zum T-Substituenten unterwirft und die Verbindung der allgemeinen Formel I gemäß Ausführungsform 1 mit den angegebenen Bedeutungen gewinnt.

52. Verfahren nach Ausführungsform 51, bei dem man die Halogenierung in Gegenwart von $C_5Cl_5$NF-triflat, $SO_2Cl_2$, NBS und IC1 durchführt.

53. (Schema 8) Verfahren zur Herstellung einer Verbindung der allgemeinen Formel I gemäß Ausführungsform 1 (Typ 30) mit R = $OR^1$, $R^1$ = H, S = H, T = $OR^4$, $R^4$ = H, U = $NO^2$, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl, insbesondere $C_{1-6}$-Alkyl, Alkenyl, insbesondere $C_{2-6}$-Alkenyl, Aryl oder Heteroaryl, Y = freies Elektronenpaar und Z = $CH_3$, bei dem man

(i) eine Ausgangsverbindung (Typ 1, 2, 3, 4, 5 oder 6) gemäß Ausführungsform 3 oder
(ii) ein Produkt eines Verfahrens gemäß Ausführungsform 15 (Typ 13) einer Nitrierung in ortho-Stellung zum T-Substituenten unterwirft und die Verbindung der allgemeinen Formel I gemäß Ausführungsform 1 mit den angegebenen Bedeutungen gewinnt.

54. Verfahren nach Ausführungsform 53, bei dem man die Nitrierung mit einem Alkalimetallnitrit, insbesondere Natriumnitrit, und Essigsäure in Gegenwart eines organischen Lösungsmittels durchführt, insbesondere Ethanol.

55. (Schema 8) Verfahren zur Herstellung einer Verbindung der allgemeinen Formel I gemäß Ausführungsform 1 (Typ 31) mit R = $OR^1$, $R^1$ = H, S = H, T = $OR^4$ , $R^4$ = H, U = $NH_2$ , V = $OR^7$, $R^7$ = $COR^8$ , $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl, insbesondere $C_{1-6}$-Alkyl, Alkenyl, insbesondere $C_{2-6}$-Alkenyl, Aryl oder Heteroaryl, Y = freies Elektronenpaar und Z = $CH_3$ , bei dem man ein Produkt eines Verfahrens gemäß Ausführungsform 53 oder 54 (Typ 30) einer katalytischen Reduktion unterwirft und die Verbindung der allgemeinen Formel I gemäß Ausführungsform 1 mit den angegebenen Bedeutungen gewinnt.

56. Verfahren nach Ausführungsform 55, bei dem man mit elementarem Wasserstoff in Gegenwart von Palladium/Aktivkohle reduziert, insbesondere in einem organischen Lösungsmittel, vorzugsweise Ethanol.

57. Schema 8) Verfahren zur Herstellung einer Verbindung der allgemeinen Formel I gemäß Ausführungsform 1 (Typ 31) mit R = $OR^1$, $R^1$ = H, S = H, T = $OR^4$, $R^4$ = H, U = $NH_2$, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl, vorzugsweise $C_{1-6}$-Alkyl, Alkenyl, insbesondere $C_{2-6}$-Alkenyl, Aryl oder Heteroaryl, Y = freies Elektronenpaar und Z = $CH_3$ , bei dem man

(a) in einer ersten Stufe

(i) eine Ausgangsverbindung (Typ 1, 2, 3, 4, 5 oder 6) gemäß Ausführungsform 3 oder
(ii) ein Produkt eines Verfahrens gemäß Ausführungsform 15 (Typ 13) einem Verfahren gemäß Ausführungsform 53 oder 54 unterwirft und

(b) in einer zweiten Stufe das erhaltene Produkt (Typ 30) einem Verfahren gemäß Ausführungsform 55 oder 56 unterwirft und die Verbindung der allgemeinen Formel I gemäß Ausführungsform 1 mit den angegebenen

Bedeutungen gewinnt.

58. (Schema 8) Verfahren zur Herstellung einer Verbindung der allgemeinen Formel I gemäß 1 (Typ 32) mit R = $OR^1$, $R^1$ = H, S = H, T = $OR^4$, $R^4$ = H, U = $NHR^3$, $R^3$ = Alkyl-CO, insbesondere $C_{1-4}$-Alkyl-CO, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl, insbesondere $C_{1-6}$-Alkyl, Alkenyl, insbesondere $C_{2-6}$-Alkenyl, Aryl oder Heteroaryl, Y = freies Elektronenpaar und Z = $CH_3$, bei dem man ein Produkt eines Verfahrens gemäß Ausführungsform 55, 56 oder 57 (Typ 31) einer Alkylierung unterwirft und die Verbindung der allgemeinen Formel I gemäß Ausführungsform 1 mit den angegebenen Bedeutungen gewinnt.

59. Verfahren nach Ausführungsform 58, bei dem man mit einem Säureanhydrid der Formel $(R^3)_2O$ mit $R^3$ = Alkyl-CO, insbesondere $C_{1-4}$-Alkyl-CO alkyliert.

60. (Schema 8) Verfahren zur Herstellung einer Verbindung der allgemeinen Formel I gemäß Ausführungsform 1 (Typ 32) mit R = $OR^1$, $R^1$ = H, S = H, T = $OR^4$, $R^4$ = H, U = $NHR^3$, $R^3$ = Alkyl-CO, insbesondere $C_{1-4}$-Alkyl-CO, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl, insbesondere $C_{1-6}$-Alkyl, Alkenyl, insbesondere $C_{2-6}$-Alkenyl, Aryl oder Heteroaryl, bei dem man

(a) in einer fakultativen ersten Stufe

(i) eine Ausgangsverbindung (Typ 1, 2, 3, 4, 5 oder 6) gemäß Ausführungsform 3 oder
(ii) ein Produkt eines Verfahrens gemäß Ausführungsform 15 (Typ 13) einem Verfahren gemäß Ausführungsform 53 oder 54 unterwirft,

(b) in einer zweiten Stufe das erhaltene Produkt (Typ 30) einem Verfahren gemäß Ausführungsform 55 oder 56 unterwirft und
(c) in einer dritten Stufe ein Verfahren gemäß Ausführungsform 58 oder 59 durchführt und

die Verbindung der allgemeinen Formel I gemäß Ausführungsform 1 mit den angegebenen Bedeutungen gewinnt.

61. (Schema 9) Verfahren zur Herstellung einer Verbindung der allgemeinen Formel I gemäß Ausführungsform 1 (Typ 33) mit R = $OR^1$, $R^1$ = H, S = U = H, T = $OR^4$, $R^4$ = H, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl, insbesondere $C_{1-6}$-Alkyl, Alkenyl, insbesondere $C_{2-6}$-Alkenyl, Aryl oder Heteroaryl, Y = O und Z = $CH_3$ , bei dem man

(i) eine Ausgangsverbindung (Typ 1, 2, 3, 4, 5 oder 6) gemäß Ausführungsform 3 oder
(ii) ein Produkt eines Verfahrens gemäß Ausführungsform 15 (Typ 13) einer Reaktion zur Bildung eines N-Oxids unterwirft und die Verbindung der allgemeinen Formel I gemäß Ausführungsform 1 mit den angegebenen Bedeutungen gewinnt.

62. Verfahren nach Ausführungsform 61, bei dem man die N-Oxid-Bildung mit m-CPBA in einem organischen Lösungsmittel durchführt, insbesondere Methylenchlorid.

63. (Schema 9) Verfahren zur Herstellung einer Verbindung der allgemeinen Formel I gemäß Ausführungsform 1 (Typ 34) mit R = $OR^1$, $R^1$ = H, S = U = H, T = $OR^4$, $R^4$ = H, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl, insbesondere $C_{1-6}$-Alkyl, Alkenyl, insbesondere $C_{2-6}$-Alkenyl, Aryl oder Heteroaryl, Y = freies Elektronenpaar, Z = $COR^{11}$ und $R^{11}$ = Alkyl, vorzugsweise $C_{1-4}$-Alkyl, insbesondere Methyl, bei dem man das Produkt eines Verfahrens gemäß Ausführungsform 61 oder 62 (Typ 33) mit einem Acylierungsmittel umsetzt und die Verbindung der allgemeinen Formel I gemäß Ausführungsform 1 mit den angegebenen Bedeutungen gewinnt.

64. Verfahren nach Ausführungsform 63, bei dem man die Acylierung mit einem Säureanhydrid durchführt, insbesondere Essigsäureanhydrid, vorzugsweise bei erhöhter Temperatur.

65. (Schema 9) Verfahren zur Herstellung einer Verbindung der allgemeinen Formel I gemäß Ausführungsform 1 (Typ 34) mit R = $OR^1$, $R^1$ = H, S = U = H, T = $OR^4$ , $R^4$ = H, V = $OR^7$, $R^7$ = $COR^8$ , $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl, insbesondere $C_{1-6}$-Alkyl, Alkenyl, insbesondere $C_{2-6}$-Alkenyl, Aryl oder Heteroaryl, Y = freies Elektronenpaar, Z = $COR^{11}$ und $R^{11}$ = Alkyl, vorzugsweise

$C_{1-4}$-Alkyl, insbesondere Methyl, bei dem man

(a) in einer ersten Stufe

(i) eine Ausgangsverbindung (Typ 1, 2, 3, 4, 5 oder 6) gemäß Ausführungsform 3 oder
(ii) ein Produkt eines Verfahrens gemäß Ausführungsform 15 (Typ 13) einem Verfahren gemäß Ausführungsform 61 oder 62 unterwirft und

(b) in einer zweiten Stufe das erhaltene Produkt (Typ 33) einem Verfahren gemäß Ausführungsform 63 oder 64 unterwirft und

die Verbindung der allgemeinen Formel I gemäß Ausführungsform 1 mit den angegebenen Bedeutungen gewinnt.

66. Therapeutisches Mittel, insbesondere Cytostatikum, mit ein oder mehreren Verbindungen gemäß Ausführungsform 1 oder 2 als Wirkstoff neben einem oder mehreren fakultativen üblichen Trägern und/oder einem oder mehreren fakultativen üblichen Verdünnungsmitteln.

67. Therapeutisches Mittel, insbesondere Cytostatikum, mit r einem oder mehreren Produkten eines Verfahrens gemäß einer der Ausführungsform en 3 bis 65 als Wirkstoff neben einem oder mehreren fakultativen üblichen Trägern und/oder einem oder mehreren fakultativen üblichen Verdünnungsmitteln.

68. Verbindung nach Ausführungsform 1 oder 2, wobei
Alkyl verzweigtes, unverzweigtes oder cyclisches $C_{1-20}$-Alkyl, insbesondere $C_{1-7}$-Alkyl, vorzugsweise $C_{1-6}$-Alkyl und besonders bevorzugt $C_{1-4}$-Alkyl ist, insbesondere Methyl, Ethyl, Propyl, Isopropyl, n-Butyl, iso-Butyl, sek-Butyl, tert-Butyl, und wobei Cycloalkyl vorzugsweise 3 bis 8 C-Atome im Ring besitzt.

69. Verbindung nach Ausführungsform 1, 2 oder 68, wobei
Alkenyl verzweigtes, unverzweigtes oder cyclisches $C_{2-20}$-Alkenyl, insbesondere $C_{2-7}$-Alkenyl, vorzugsweise $C_{2-6}$-Alkenyl und besonders bevorzugt $C_{2-4}$-Alkenyl ist, insbesondere Vinyl, Allyl, Propen-1-yl, Propen-2-yl, But-1-en-1-yl, But-1-en-2-yl, But-1-en-3-yl, But-1-en-4-yl, But-2-en-1-yl, But-2-en-2-yl, 2-Methyl-propen-1-yl, 2-Methyl-propen-3-yl ist, und wobei Cycloalkenyl vorzugsweise 3 bis 8 C-Atome im Ring besitzt, und die Anzahl der Doppelbindungen der Alkenylgruppen 1 bis 3 beträgt.

70. Verbindung nach Ausführungsform 1, 2, 68 oder 69, wobei Aryl Phenyl, Naphthyl oder Biphenylyl bedeutet.

71. Verbindung nach Ausführungsform 1, 2, 68, 69, oder 70, wobei Heteroaryl Furyl, Thienyl, Imidazolyl, Indolyl, Pyridyl, Pyridinyl, Pyrrolyl, Thiazolyl, Oxazolyl, oder Pyrimidinyl bedeutet.

72. Verbindung nach Ausführungsform 1, 2, 68, 69, 70 oder 71, wobei Alkyl, Alkenyl, Aryl und Heteroaryl unsubstituiert oder substituiert sind und insbesondere in beliebiger Position 1 bis 3 Substituenten aus der durch $C_{1-3}$-Alkyl, $C_{1-3}$-Alkyloxy, Hydroxy, Amino ($NH_2$) oder Nitro ($NO_2$) gebildeten Gruppen tragen.

## Patentansprüche

**1.** Verbindung der folgenden allgemeinen Formel I (Tubulysin):

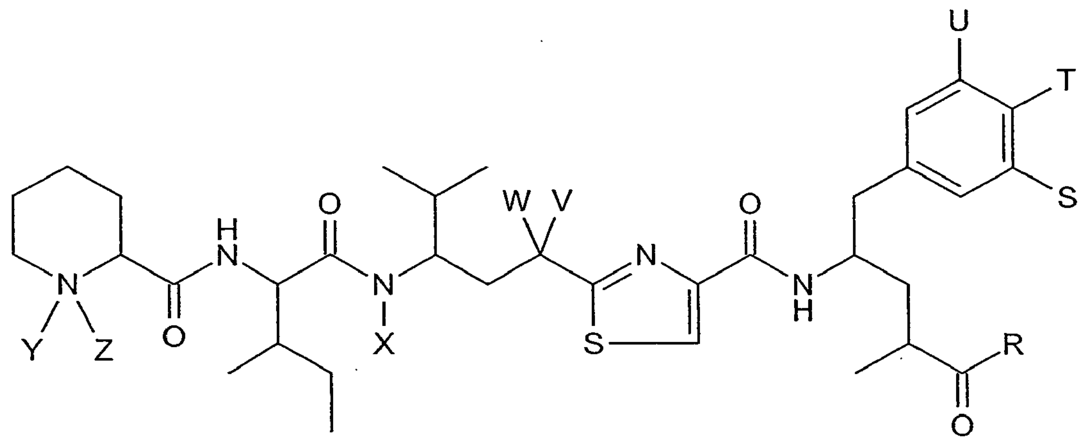

mit den folgenden Bedeutungen für R, $R^1$, $R^2$, $R^3$, $R^4$, $R^5$, $R^6$, $R^7$, $R^8$, $R^{11}$, S, T, U, V, W, X, Y und Z:
R = H, Alkyl, Aryl, $OR^1$, $NR^1R^2$ oder

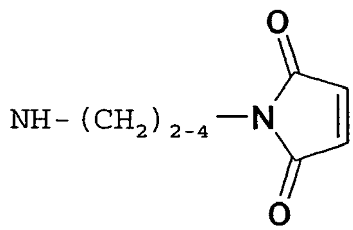

$R^1$ = H, Alkyl oder Aryl
$R^2$ = H, Alkyl oder Aryl
S = H, Hal, $NO_2$ oder $NHR^3$
U = H, Hal, $NO_2$ oder $NHR^3$
$R^3$ = H, HCO oder Alkyl-CO
T = H oder $OR^4$
$R^4$ = H, Alkyl, Aryl, $COR^5$, P(O) $(OR^6)_2$ oder $SO_3R^6$
$R^5$ = Alkyl, Alkenyl, Aryl oder Heteroaryl
$R^6$ = H, Alkyl oder Metallion
V = H, $OR^7$, Hal oder (mit W = O) O
$R^7$ = H, Alkyl oder $COR^8$
$R^8$ = Alkyl, Alkenyl oder Aryl
W = H oder Alkyl oder (mit V) O
X = H, Alkyl oder Alkenyl
Y = = (für Z = $CH_3$ oder $COR^{11}$) freies Elektronenpaar oder (für Z = $CH_3$) O
$R^{11}$ = Alkyl, $CF_3$ oder Aryl und/oder
Z = (für Y = O oder freies Elektronenpaar) $CH_3$ oder (für Y = freies Elektronenpaar) $COR^{11}$.

**2.** Verbindung nach Anspruch 1 mit
R, $R^1$, $R^4$, $R^5$, $R^8$, und/oder $R^{11}$ = unsubstituiertes oder substituiertes Phenyl, insbesondere $C_{1-4}$-Alkyl-substituiertes Phenyl
$R^5$ = $C_{1-4}$-Alkyl, $C_{2-6}$-Alkenyl oder Pyridyl
$R^5$ und/oder X = $C_{2-4}$-Alkenyl
$R^6$ = Alkalimetall-Ion, insbesondere Na-Ion, oder Erdalkalimetall-Ion
$R^8$ = $C_{2-4}$-Alkenyl

**3.** Therapeutisches Mittel, insbesondere Cytostatikum, mit ein oder mehreren Verbindungen gemäß Anspruch 1 oder 2 als Wirkstoff neben einem oder mehreren fakultativen üblichen Trägern und/oder einem oder mehreren fakultativen

üblichen Verdünnungsmitteln.

**4.** Verbindung nach Anspruch 1 oder 2, wobei
Alkyl verzweigtes, unverzweigtes oder cyclisches $C_{1-20}$-Alkyl, insbesondere $C_{1-7}$-Alkyl, vorzugsweise $C_{1-6}$-Alkyl und besonders bevorzugt $C_{1-4}$-Alkyl ist, insbesondere Methyl, Ethyl, Propyl, Isopropyl, n-Butyl, iso-Butyl, sek-Butyl, tert-Butyl, und wobei Cycloalkyl vorzugsweise 3 bis 8 C-Atome im Ring besitzt.

**5.** Verbindung nach Anspruch 1, 2 oder 4, wobei
Alkenyl verzweigtes, unverzweigtes oder cyclisches $C_{2-20}$-Alkenyl, insbesondere $C_{2-7}$-Alkenyl, vorzugsweise $C_{2-6}$-Alkenyl und besonders bevorzugt $C_{2-4}$-Alkenyl ist, insbesondere Vinyl, Allyl, Propen-1-yl, Propen-2-yl, But-1-en-1-yl, But-1-en-2-yl, But-1-en-3-yl, But-1-en-4-yl, But-2-en-1-yl, But-2-en-2-yl, 2-Methyl-propen-1-yl, 2-Methyl-propen-3-yl ist, und wobei Cycloalkenyl vorzugsweise 3 bis 8 C-Atome im Ring besitzt, und die Anzahl der Doppelbindungen der Alkenylgruppen 1 bis 3 beträgt.

**6.** Verbindung nach Anspruch 1, 2, 4 oder 5, wobei
Aryl Phenyl, Naphthyl oder Biphenylyl bedeutet.

**7.** Verbindung nach Anspruch 1, 2, 4, 5, oder 6, wobei Heteroaryl Furyl, Thienyl, Imidazolyl, Indolyl, Pyridyl, Pyridinyl, Pyrrolyl, Thiazolyl, Oxazolyl, oder Pyrimidinyl bedeutet.

**8.** Verbindung nach Anspruch 1, 2, 4, 5, 6 oder 7, wobei
Alkyl, Alkenyl, Aryl und Heteroaryl unsubstituiert oder substituiert sind und insbesondere in beliebiger Position 1 bis 3 Substituenten aus der durch $C_{1-3}$-Alkyl, $C_{1-3}$-Alkyloxy, Hydroxy, Amino ($NH_2$) oder Nitro ($NO_2$) gebildeten Gruppen tragen.

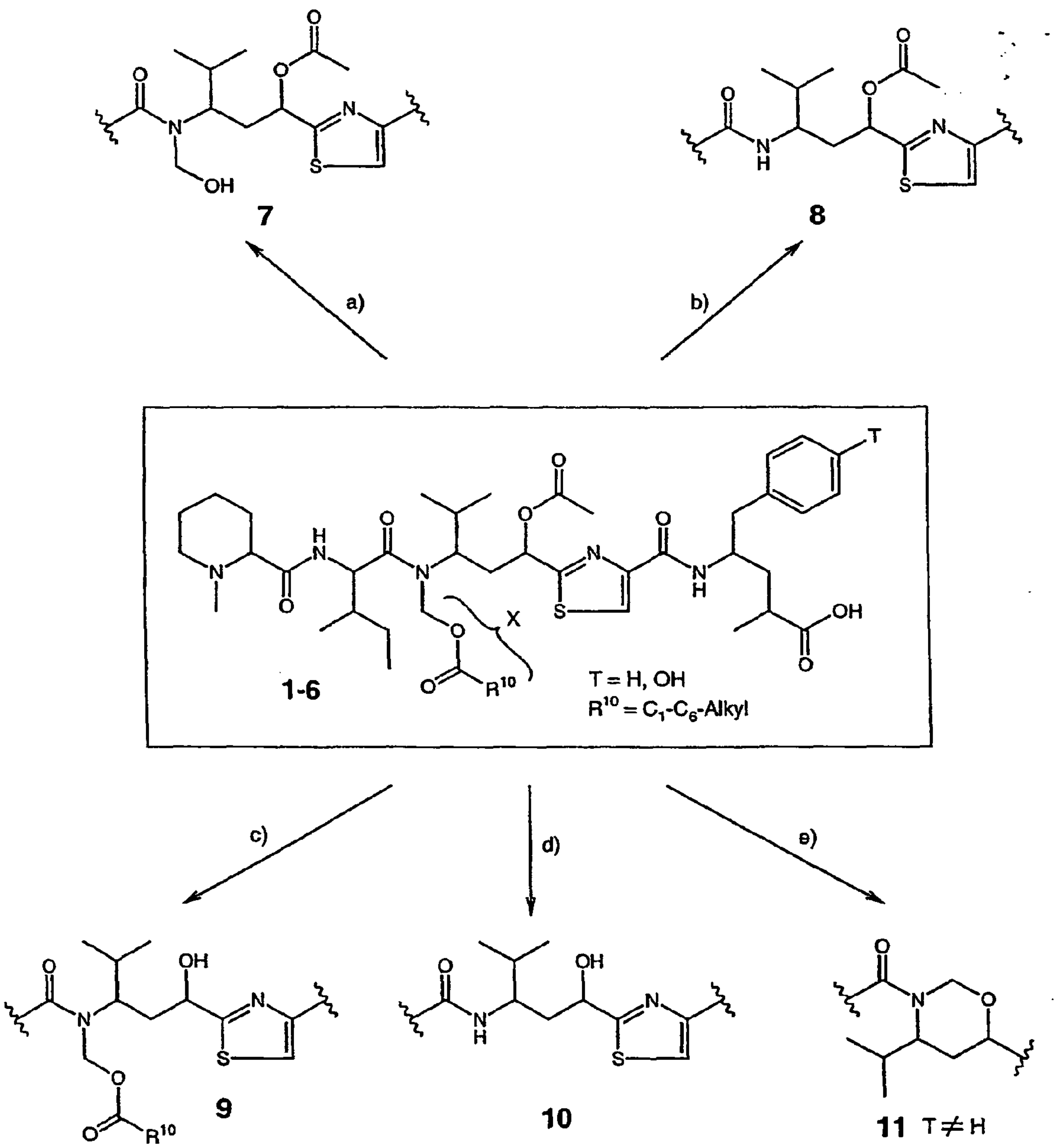


a) 0.1 M HCl, Dioxan, 50°C; b) 0.1 M HCl, 100°C; c) $NH_3$, MeOH; d) 1 M NaOH, MeOH; e) 0.5 M HCl, 100°C

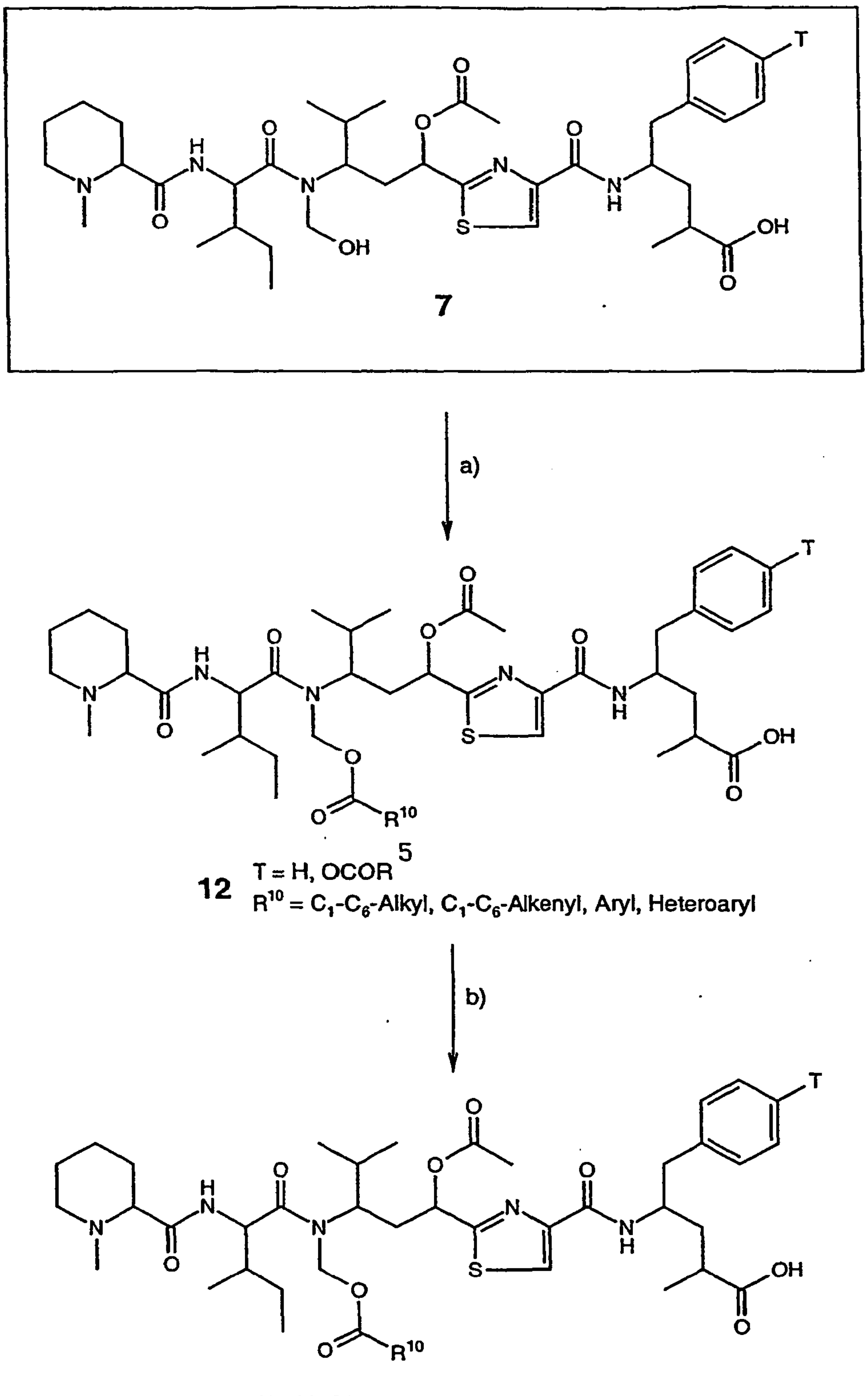


7

12 T = H, OCOR5
$R^{10}$ = $C_1$-$C_6$-Alkyl, $C_1$-$C_6$-Alkenyl, Aryl, Heteroaryl

13 T = H, OH
$R^{10}$ = $C_1$-$C_6$-Alkyl, $C_1$-$C_6$-Alkenyl, Aryl, Heteroaryl

a) $R^{10}COCl$, $Et_3N$; b) $NH_3$

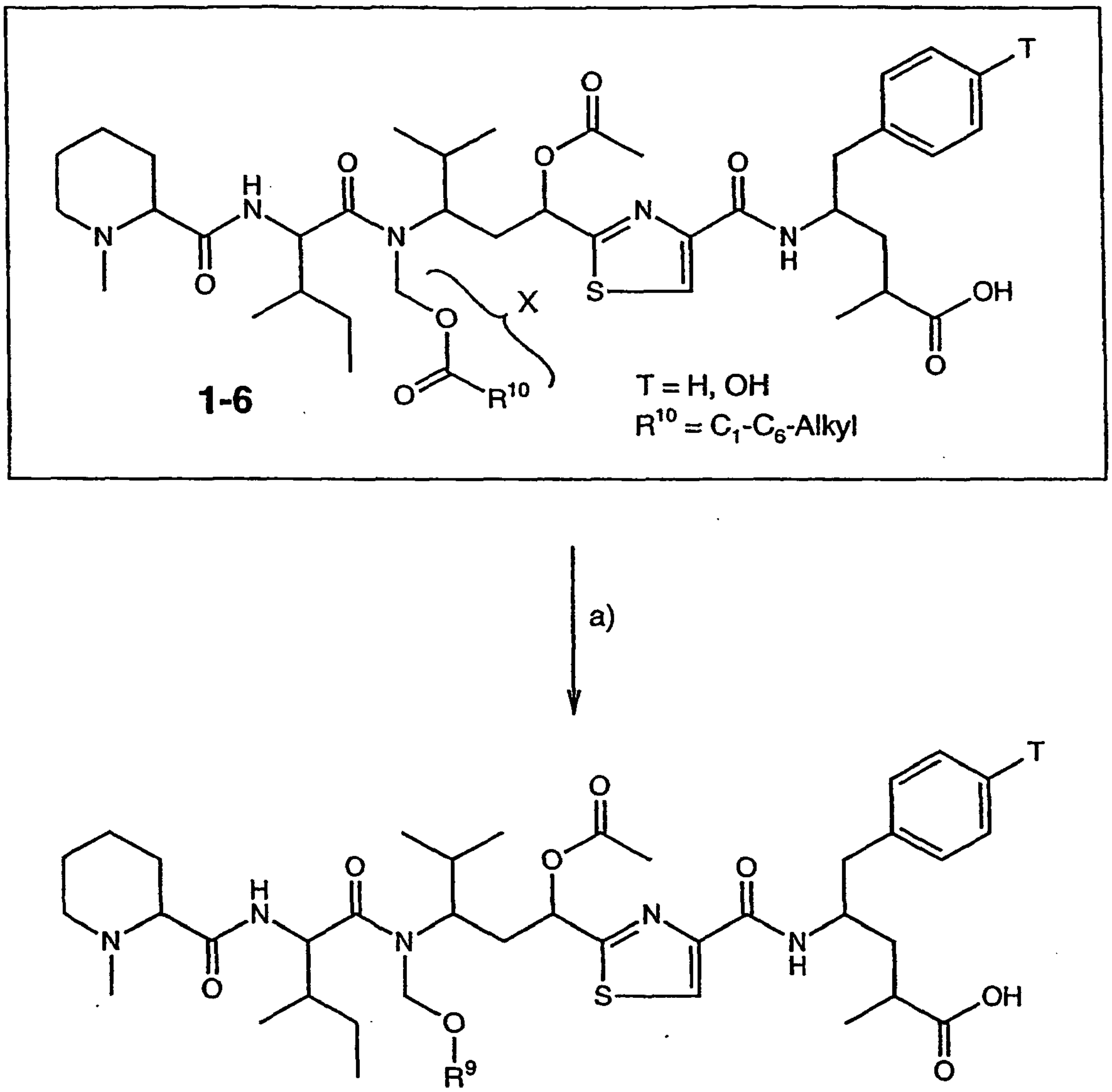


**14** $R^9$ = $C_1$-$C_4$-Alkyl, Alkenyl, Aryl

a) *p*-$CH_3$-$C_6H_4SO_2OH$, $R^9OH$, THF, 80°C

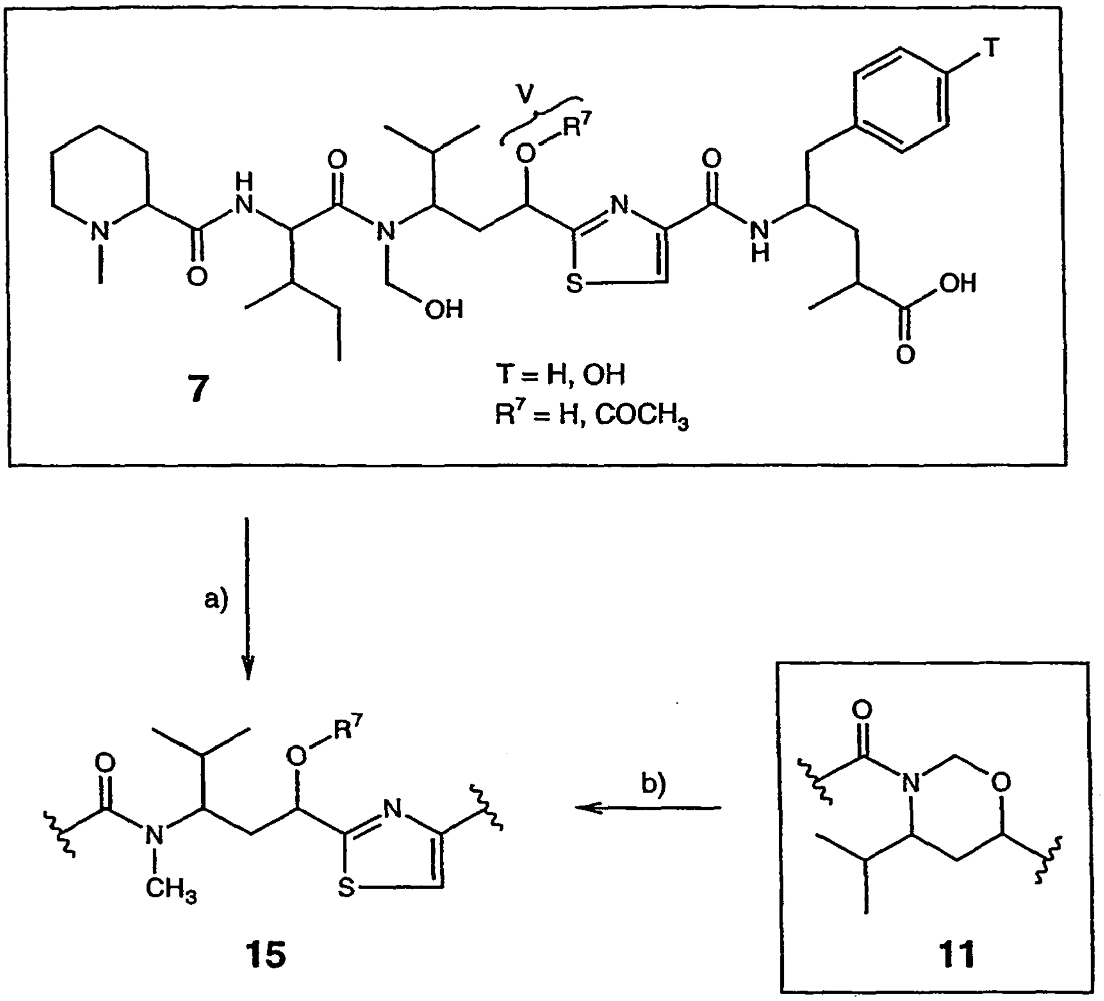


a) $NaCNBH_3$, TFA, MeOH; b) $NaCNBH_3$, $Me_3SiCl$, $CH_3CN$

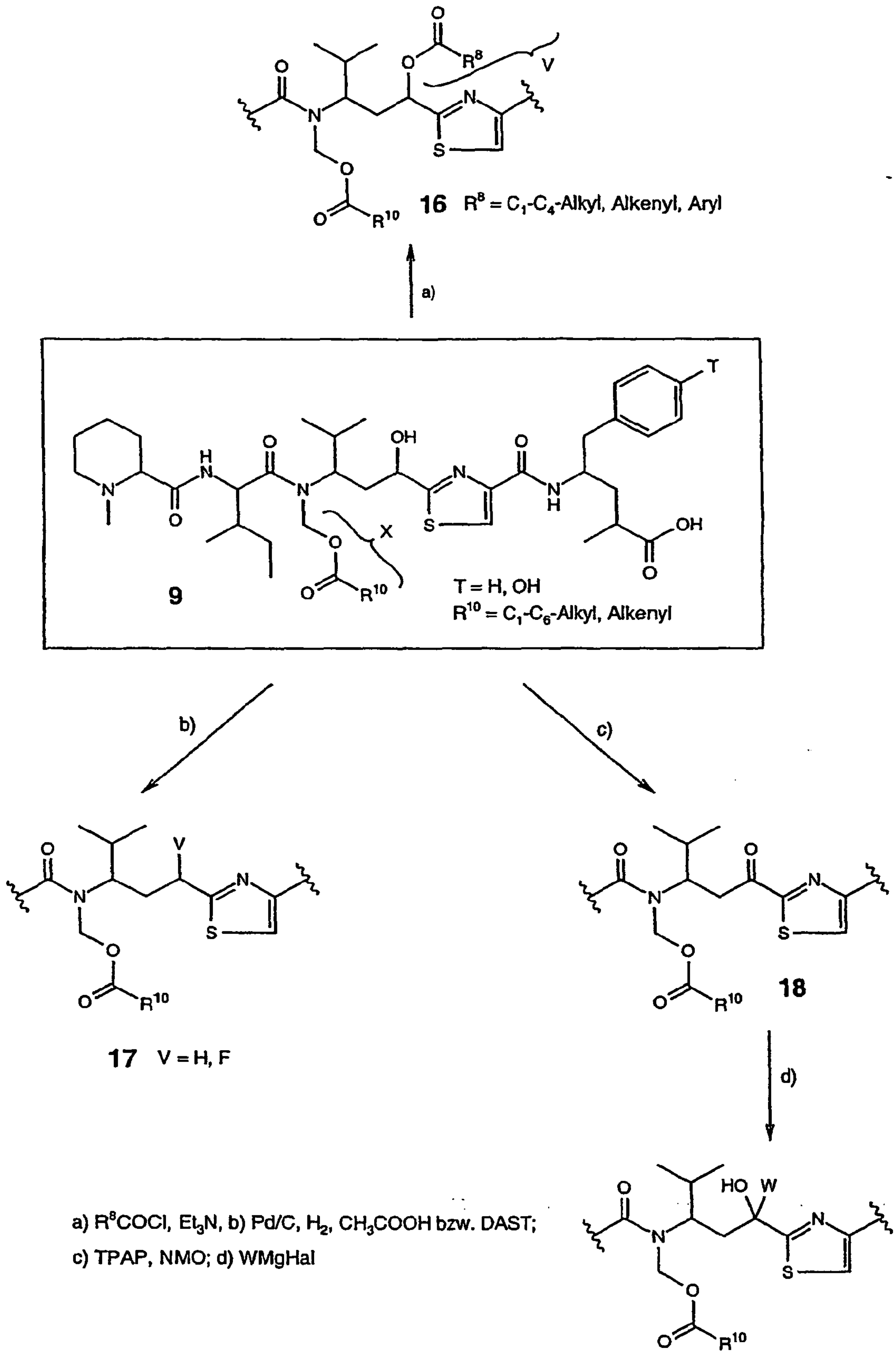


16 $R^8 = C_1$-$C_4$-Alkyl, Alkenyl, Aryl

9 T = H, OH
$R^{10} = C_1$-$C_6$-Alkyl, Alkenyl

17 V = H, F

18

19 W = $C_1$-$C_4$-Alkyl

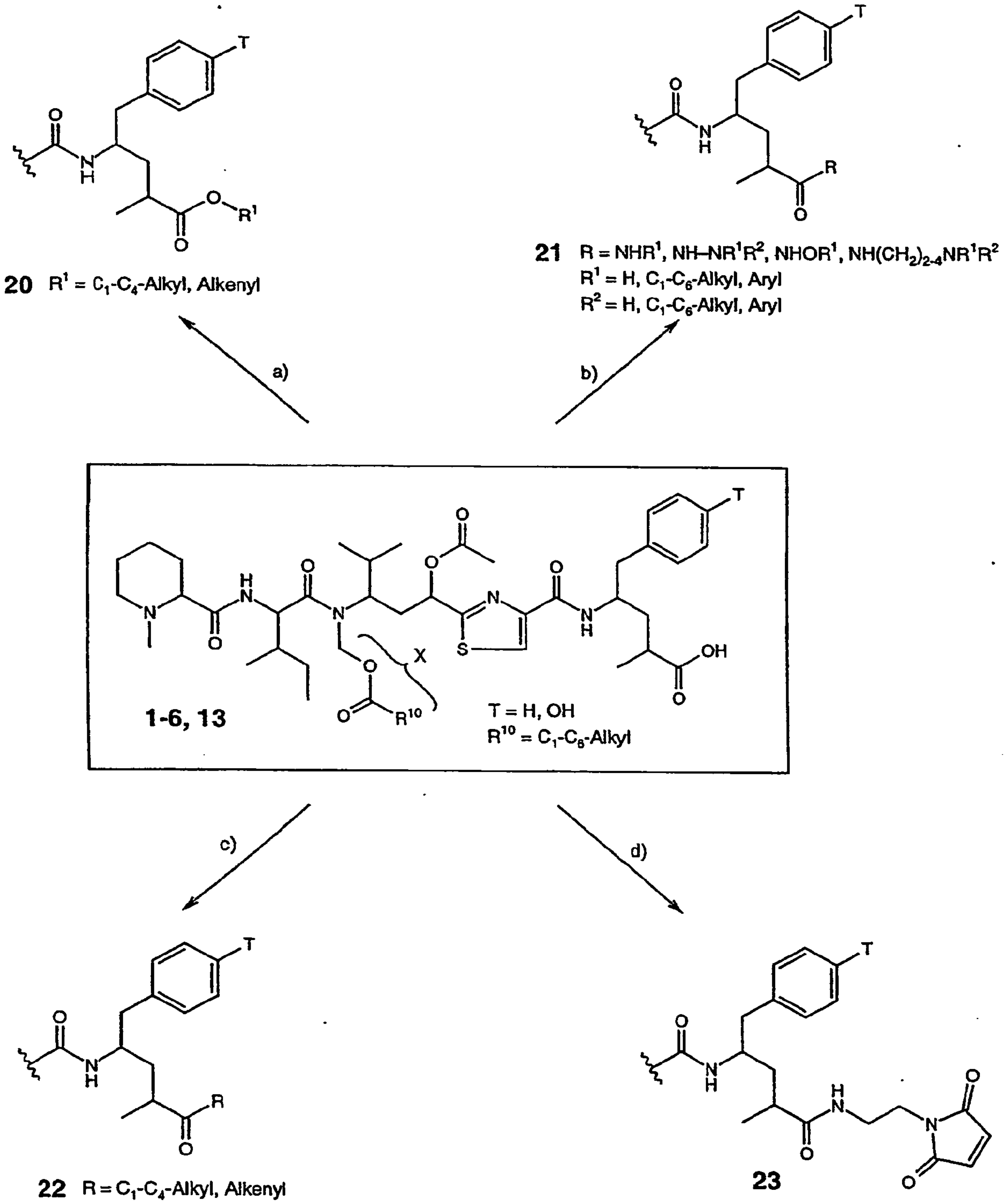


**20** $R^1 = C_1$-$C_4$-Alkyl, Alkenyl

**21** R = $NHR^1$, $NH$–$NR^1R^2$, $NHOR^1$, $NH(CH_2)_{2\text{-}4}NR^1R^2$
$R^1$ = H, $C_1$-$C_6$-Alkyl, Aryl
$R^2$ = H, $C_1$-$C_6$-Alkyl, Aryl

**1-6, 13**

T = H, OH
$R^{10} = C_1$-$C_6$-Alkyl

**22** R = $C_1$-$C_4$-Alkyl, Alkenyl

**23**

a) EDC, $R^1OH$, DMAP, $CH_2Cl_2$; b) EDC, RH, $CH_2Cl_2$ oder *i*-Butylchlorformiat, $Et_3N$, RH, abs. THF
c) RLi; d) EDC, 1-(2-Aminoethyl)-pyrrol-2,5-dion,$CH_2Cl_2$

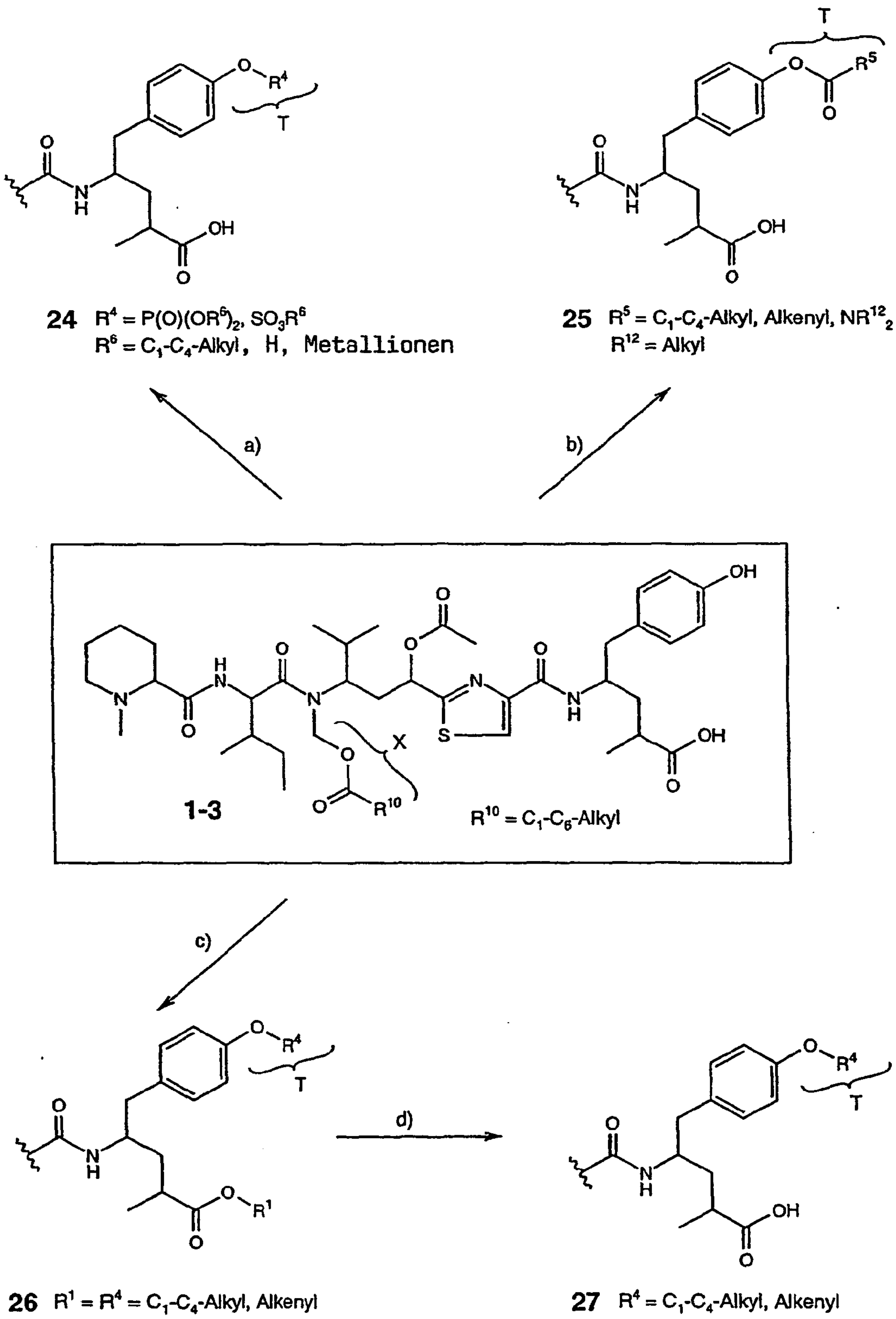


**24** $R^4 = P(O)(OR^6)_2$, $SO_3R^6$
$R^6 = C_1$-$C_4$-Alkyl, H, Metallionen

**25** $R^5 = C_1$-$C_4$-Alkyl, Alkenyl, $NR^{12}_2$
$R^{12}$ = Alkyl

**1-3** $R^{10} = C_1$-$C_6$-Alkyl

**26** $R^1 = R^4 = C_1$-$C_4$-Alkyl, Alkenyl

**27** $R^4 = C_1$-$C_4$-Alkyl, Alkenyl

a) $P(O)(OR^6)_2OH$, $I_2$, Pyridin, $CH_2Cl_2$ bzw. Pyridin-$SO_3$; b) $R^5COCl$, $Et_3N$, abs. THF;
c) $Ag_2O$, $R^4I$, $CH_2Cl_2$; für $R^4 = CH_3$: $CH_2N_2$, MeOH; d) Schweineleber-Esterase, $KH_2PO_4$-Puffer, 36°C;

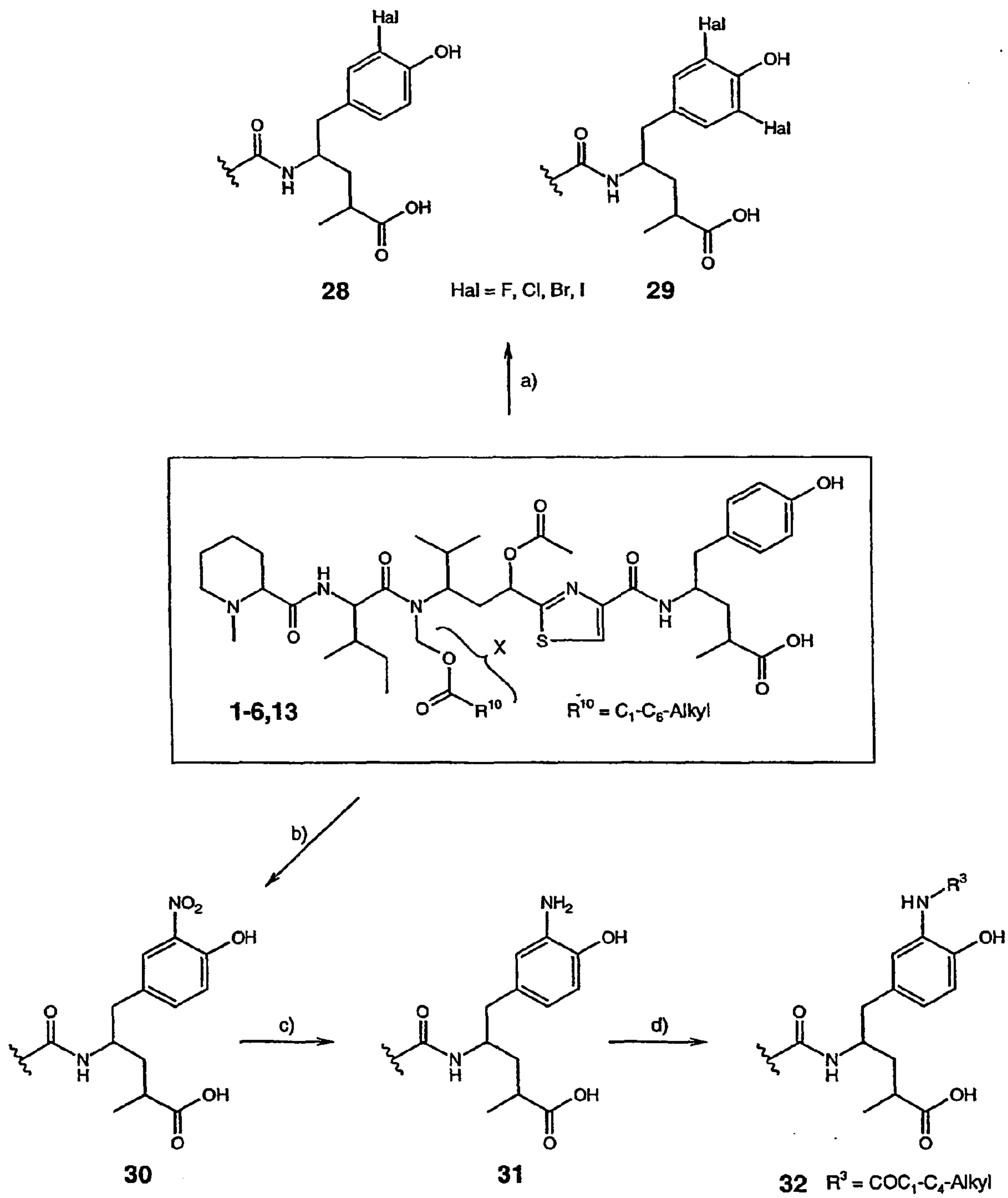


28 Hal = F, Cl, Br, I 29

1-6,13 $R^{10} = C_1$-$C_6$-Alkyl

30 31 32 $R^3 = COC_1$-$C_4$-Alkyl

a) $C_5Cl_5NF$-triflat, $SO_2Cl_2$, NBS, ICl; b) $NaNO_2$, $CH_3COOH$, EtOH; c) Pd/C, $H_2$, EtOH; d) $(R^3CO)_2O$

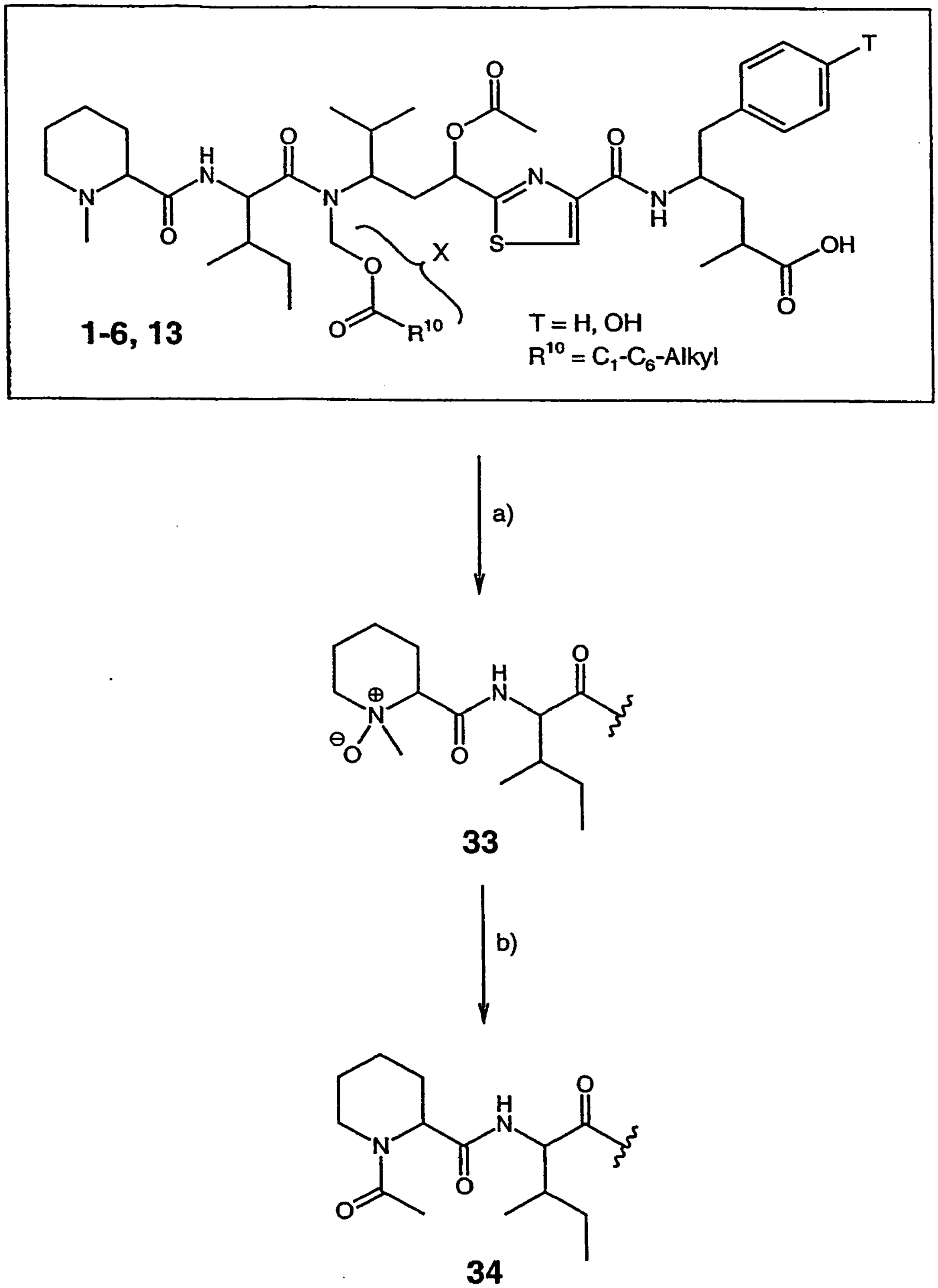


**1-6, 13**

**33**

**34**

a) *m*-CPBA, $CH_2Cl_2$; b) $Ac_2O$, 75°C

Europäisches Patentamt
European Patent Office
Office européen des brevets

# EUROPÄISCHER RECHERCHENBERICHT

Nummer der Anmeldung
EP 08 01 8687

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 196 38 870 A1 (BIOTECHNOLOG FORSCHUNG GMBH [DE]) 26. März 1998 (1998-03-26)<br>* Ansprüche 1-10; Abbildungen 1-3 *<br>----- | 1-8 | INV.<br>C07K5/02<br>C07D207/26<br>C07D277/56<br>C07D417/12<br>C07F7/18<br>C07C271/22<br>C07D211/60<br>A61K38/08 |
| X | DE 100 08 089 A1 (BIOTECHNOLOG FORSCHUNG GMBH [DE]) 31. Oktober 2001 (2001-10-31)<br>* Seite 2; Anspruch 1 *<br>----- | 1-8 | |
| E | WO 2004/005327 A (MORPHOCHEM AKTIENGESELLSCHAFT FUER KOMBINATORISCHE; DOEMLING, ALEXANDE)<br>15. Januar 2004 (2004-01-15)<br>* Ansprüche 1-18 *<br>----- | 1-8 | |
| E | WO 2008/106080 A (UNIV PITTSBURGH [US]; WIPF PETER [US]; WANG ZHIYONG [US] UNIV PITTSBUR) 4. September 2008 (2008-09-04)<br>* Ansprüche 1-39,41-54; Tabelle 1 *<br>----- | 1-8 | |
| P,X | WANG Z ET AL.: "Structure-activity and High-content Imaging Analyses for Novel Tubulysins"<br>CHEMICAL BIOLOGY & DRUG DESIGN,<br>Bd. 70, 2007, Seiten 75-86, XP002509547<br>* das ganze Dokument, insbesondere Schema 1, Schema 2 und Abbildungen 2 bis 6 *<br>----- | 1-8 | RECHERCHIERTE SACHGEBIETE (IPC)<br>C07K<br>C07D<br>A61K |
| P,X | DÖMLING A ET AL: "Total synthesis of Tubulysin U and V"<br>ANGEWANDTE CHEMIE. INTERNATIONAL EDITION, DEVCH VERLAG, WEINHEIM,<br>Bd. 45, Nr. 43,<br>6. November 2006 (2006-11-06), Seiten 7235-7239, XP002505480<br>* Seite 7328; Tabelle 1 *<br>----- | 1-8 | |

Der vorliegende Recherchenbericht wurde für alle Patentansprüche erstellt

3

| Recherchenort | Abschlußdatum der Recherche | Prüfer |
|---|---|---|
| Den Haag | 8. Januar 2009 | Schmidt, Harald |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 08 01 8687

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

08-01-2009

| Im Recherchenbericht angeführtes Patentdokument | Datum der Veröffentlichung | Mitglied(er) der Patentfamilie | Datum der Veröffentlichung |
|---|---|---|---|
| DE 19638870 A1 | 26-03-1998 | AU 4555097 A | 17-04-1998 |
| | | WO 9813375 A1 | 02-04-1998 |
| DE 10008089 A1 | 31-10-2001 | KEINE | |
| WO 2004005327 A | 15-01-2004 | AU 2003266233 A1 | 23-01-2004 |
| | | EP 1523493 A1 | 20-04-2005 |
| | | US 2005239713 A1 | 27-10-2005 |
| WO 2008106080 A | 04-09-2008 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 19638870 A1


### In der Beschreibung aufgeführte Nicht-Patentliteratur


- **F. SASSE ; H. STEINMETZ ; J. HEIL ; G. HÖFLE ; H. REICHENBACH.** *J. Antibiot.,* 2000, vol. 53, 579-558